# EUROPEAN PATENT APPLICATION

(11) **EP 4 741 497 A1**
(43) Date of publication of application: **13.05.2026**
(21) Application number: 24870716.8
(22) Date of filing: 24.09.2024
(51) Int. Cl.: C12N 9/22, C07K 19/00, C12N 15/113, C12N 15/55, C12N 15/62, C12N 15/85, C12N 15/65

(54) **NOVEL CRISPR ENZYME, AND SYSTEM AND APPLICATION**

(30) Priority: 25.09.2023 CN 202311235588
(71) Applicant: Shandong Shunfeng Biotechnology Co., Ltd., Jinan, Shandong 250000 (CN)
(72) Inventor: DUAN, Zhiqiang, Jinan, Shandong 250000 (CN); LI, Shanshan, Jinan, Shandong 250000 (CN); LIU, Ruiheng, Jinan, Shandong 250000 (CN)
(74) Representative: Bayramoglu et al.
(86) International application number: PCT/CN2024/120792
(87) International publication number: WO 2025/067172

(57) **Abstract**

The present disclosure provides a clustered regularly interspaced short palindromic repeat (CRISPR)-associated (Cas) enzyme, and belongs to the field of nucleic acid editing, and particularly to the technical field of CRISPR. The present disclosure has promising application prospects in the field of gene editing.

## Description

The present application claims priority to Chinese Patent Application CN202311235588.4, filed on September 25, 2023, which is incorporated herein by reference in its entirety.

### TECHNICAL FIELD

The present disclosure relates to the field of gene editing, and in particular to the technical field of clustered regularly interspaced short palindromic repeats (CRISPR). Specifically, the present disclosure finds a novel CRISPR-associated (Cas) enzyme and develops a corresponding gene editing tool based on the novel Cas enzyme, and a use thereof.

### BACKGROUND TECHNOLOGY

CRISPR/Cas technology is a widely-used gene editing technology. In CRISPR/Cas technology, the RNA guidance is used to specifically bind to a target sequence on a genome and cleave DNA to produce double strand breaks, and the site-directed gene editing is conducted through biological non-homologous end joining or homologous recombination.

The CRISPR/Cas9 system is the most common type II CRISPR system, which recognizes a protospacer adjacent motif (PAM) of 3'-NGG and enables blunt-end cleavage for a target sequence. The CRISPR/Cas Type V system is a newly discovered CRISPR system. The CRISPR/Cas Type V system has a motif of 5'-TTN and enables sticky-end cleavage for a target sequence, such as Cpf1, C2c1, CasX, and CasY. However, the various existing CRISPR/Cas systems have distinct advantages and disadvantages. For example, Cas9, C2c1, and CasX all require two RNAs for RNA guidance, while Cpf1 only requires one guide RNA (gRNA) and can be used for multiplex gene editing. CasX has a size of 980 amino acids, while common Cas9, C2c1, CasY, and Cpf1 typically have a size of about 1,300 amino acids. In addition, PAM sequences of Cas9, Cpf1, CasX, and CasY are relatively complex and diverse. C2c1 can only recognize the strict 5'-TTN. Thus, a target site of C2c1 is more easily predicted than target sites of other systems, which reduces the potential off-target effect of C2c1.

In summary, given that the currently available CRISPR/Cas systems are all constrained by some shortcomings, it is of great significance for the development of biotechnology to develop a robust novel CRISPR/Cas system with multiple favorable properties.

### CONTENT OF THE INVENTION

Through a large number of experiments and repeated explorations, the inventors of the present application have unexpectedly discovered a novel endonuclease (Cas enzyme). On the basis of this discovery, the inventors develop a novel CRISPR/Cas system, and a gene editing method and nucleic acid detection method based on the CRISPR/Cas system.

### Cas effector protein

In an aspect, the present disclosure provides a Cas protein, which is an effector protein in a CRISPR/Cas system. In the present disclosure, the Cas protein is referred to as Cas-sf6728. An amino acid sequence of the Cas-sf6728 protein is shown in SEQ ID No. 1.

In an embodiment, the Cas protein has an amino acid sequence possessing a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with an amino acid sequence shown in SEQ ID No. 1 and basically retains a biological function of SEQ ID No. 1. Preferably, the Cas protein is derived from the same species as that of the Cas-sf6728.

In an embodiment, the Cas protein has an amino acid sequence including substitution, deletion, or addition of one or more amino acids relative to the amino acid sequence shown in SEQ ID No. 1 and basically retains the biological function of SEQ ID No. 1. The substitution, deletion, or addition of one or more amino acids includes substitution, deletion, or addition of 1, 2, 3, 4, 5, 6, 7, 8, 9, or 10 amino acids.

In an embodiment, the Cas protein is a derivatized protein with the same biological function as a protein possessing the amino acid sequence shown in SEQ ID No.1.

In an embodiment, the Cas protein has the amino acid sequence shown in SEQ ID No. 1.

In an embodiment, the Cas protein includes a mutation of any one or more (for example, any 1, 2, 3, 4, 5, 6, 7, 8, 9, or 10) of 36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, and 344th amino acids relative to the amino acid sequence shown in SEQ ID No. 1.

In an embodiment, the Cas protein includes a mutation of 65th and 75th amino acids relative to the amino acid sequence shown in SEQ ID No. 1.

In an embodiment, an amino acid sequence of the Cas protein has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with the amino acid sequence shown in SEQ ID No. 1, and the amino acid sequence of the Cas protein includes a mutation of any one or more (for example, any 1, 2, 3, 4, 5, 6, 7, 8, 9, or 10) of36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, and 344th amino acids relative to the amino acid sequence shown in SEQ ID No. 1.

In an embodiment, the 36th amino acid is mutated to an amino acid other than T, for example, A, V, G, L, Q, F, W, Y, D, K, E, N, M, S, C, P, H, R, or I, and preferably W.

In an embodiment, the 39th amino acid is mutated to an amino acid other than D, for example, A, V, G, L, Q, F, W, Y, S, K, E, N, M, T, C, P, H, R, or I, and preferably L.

In an embodiment, the 65th or 191st amino acid is mutated to an amino acid other than L, for example, A, V, G, Y, Q, F, W, S, D, K, E, N, M, T, C, P, H, R, or I, and preferably R.

In an embodiment, the 69th or 154th or 278th amino acid is mutated to an amino acid other than S, for example, A, V, G, Y, Q, F, W, L, D, K, E, N, M, T, C, P, H, R, or I, and preferably R.

In an embodiment, the 73rd or 119th or 155th or 281st or 344th amino acid is mutated to an amino acid other than A, for example, S, V, G, Y, Q, F, W, L, D, K, E, N, M, T, C, P, H, R, or I, and preferably R.

In an embodiment, the 75th amino acid is mutated to an amino acid other than V, for example, A, D, G, L, Q, F, W, Y, S, K, E, N, M, T, C, P, H, R, or I, and preferably R.

In an embodiment, the 122nd or 195th or 264th amino acid is mutated to an amino acid other than T, for example, A, V, G, Y, Q, F, W, L, D, K, E, N, M, S, C, P, H, R, or I, and preferably R.

In an embodiment, the 132nd amino acid is mutated to an amino acid other than N, for example, A, D, G, L, Q, F, W, Y, S, K, E, V, M, T, C, P, H, R, or I, and preferably R.

In an embodiment, the 157th or 296th amino acid is mutated to an amino acid other than D, for example, A, V, G, Y, Q, F, W, S, L, K, E, N, M, T, C, P, H, R, or I, and preferably R.

In an embodiment, the 156th or 171st or 342nd amino acid is mutated to an amino acid other than Q, for example, A, V, G, Y, T, F, W, L, D, K, E, N, M, S, C, P, H, R, or I, and preferably R.

In an embodiment, the 186th or 208th amino acid is mutated to an amino acid other than E, for example, A, V, G, Y, Q, F, W, S, L, K, D, N, M, T, C, P, H, R, or I, and preferably R.

In an embodiment, the 304th amino acid is mutated to an amino acid other than H, for example, A, D, G, L, Q, F, W, Y, S, K, E, V, M, T, C, P, N, R, or I, and preferably R.

In an embodiment, corresponding to the amino acid sequence shown in SEQ ID No. 1, the Cas protein includes a mutation of any one or more (for example, any 1, 2, 3, 4, 5, 6, 7, 8, 9, or 10) of36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, and 344th amino acids relative to an amino acid sequence of a parental Cas protein.

In an embodiment, corresponding to the amino acid sequence shown in SEQ ID No. 1, the Cas protein includes a mutation of 65th and 75th amino acids relative to an amino acid sequence of a parental Cas protein.

In an embodiment, the parental Cas protein is a natural wild-type (WT) Cas protein. In other embodiments, the parental Cas protein is an engineered Cas protein.

Cas proteins from various organisms can be adopted as the parental Cas protein. In some embodiments, the parental Cas protein has a nuclease activity. In some embodiments, the parental Cas protein is a nuclease, which can cleave two strands of a target double-stranded nucleic acid (such as double-stranded DNA). In some embodiments, the parental Cas protein is a nickase, which can cleave a single strand of a target double-stranded nucleic acid (such as double-stranded DNA).

In an embodiment, the parental Cas protein is a Cas protein from a Cas12f family or a Cas14 family or a CasZ family.

In an embodiment, an amino acid sequence of the Cas protein from the Cas12f family or the Cas 14 family or the CasZ family has a sequence identity of at least 70%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% with SEQ ID No. 1.

In an embodiment, an amino acid sequence of the parental Cas protein has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with SEQ ID No. 1.

In an embodiment, the amino acid sequence of the parental Cas protein is shown in SEQ ID No. 1.

In some embodiments, the Cas protein of the present disclosure can recognize PAM, and a target nucleic acid includes or consists of the PAM.

It is clear to those skilled in the art that a structure of a protein can be changed without adversely affecting the activity and functionality of the protein. For example, one or more conservative amino acid substitutions can be introduced into an amino acid sequence of a protein without adversely affecting the activity and/or three-dimensional (3D) structure of the protein molecule. Those skilled in the art are aware of examples and implementations of the conservative amino acid substitutions. Specifically, an amino acid residue can be substituted by another amino acid residue that belongs to the same group as the amino acid residue to be substituted. That is, a nonpolar amino acid residue can be substituted by another nonpolar amino acid residue; an uncharged polar amino acid residue can be substituted by another uncharged polar amino acid residue; a basic amino acid residue can be substituted by another basic amino acid residue; and an acidic amino acid residue can be substituted by another acidic amino acid residue. Such substituted amino acid residues may be or may not be encoded by genetic codes. As long as a substitution does not result in the loss of biological activity of a protein, a conservative substitution in which an amino acid is substituted by another amino acid belonging to the same group falls within the scope of the present disclosure. Therefore, the protein of the present disclosure may include one or more conservative substitutions in the amino acid sequence, and these conservative substitutions are preferably generated according to the following table. In addition, the present disclosure also covers proteins with one or more other non-conservative substitutions, as long as the non-conservative substitutions do not significantly affect the desired function and biological activity of the protein of the present disclosure.

Conservative amino acid substitutions can be made at one or more predicted non-essential amino acid residues. Non-essential amino acid residues are amino acid residues that can be changed (deleted or substituted) without changing the biological activity, while essential amino acid residues are required for biological activity. A conservative amino acid substitution refers to a substitution in which an amino acid residue is substituted by an amino acid residue with a similar side chain. An amino acid substitution can be made in a non-conservative region of the Cas protein described above. Generally, such a substitution is not made to a conservative amino acid residue or an amino acid residue located within a conservative motif, because such a residue is required for protein activity. However, those skilled in the art should understand that a functional variant may have few conservative or non-conservative variations in conservative regions.

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

It is well known in the art that one or more amino acid residues can be changed (substituted, deleted, truncated, or inserted) at the N-terminus and/or C-terminus of a protein while still retaining the functional activity of the protein. Therefore, a protein obtained by changing one or more amino acid residues at the N-terminus and/or C-terminus of the Cas protein while retaining its desired functional activity is also within the scope of the present disclosure. The change may include a change introduced by a modern molecular method such as PCR, and the method includes PCR amplification that alters or extends a protein coding sequence by introducing an amino acid coding sequence into an oligonucleotide used in PCR amplification.

It should be recognized that a protein can be altered in various ways, including amino acid substitution, deletion, truncation, and insertion, and methods for such operations are generally known in the art. For example, amino acid sequence variants of the protein described above can be prepared through mutation of DNA. It can also be completed through other mutagenesis forms and/or through directed evolution, for example, known mutagenesis, recombination, and/or shuffling methods can be used in combination with a related screening method to achieve the substitution, deletion, and/or insertion of one or more amino acids.

Those skilled in the art can understand that these small amino acid changes in the Cas protein of the present disclosure can be naturally present (for example, natural mutations) or can be induced (for example, using r-DNA technology), which do not affect the function or activity of the protein. If these mutations occur in a catalytic domain, an active site, or another functional domain of the protein, the properties of the polypeptide may be changed, but the polypeptide may retain its activity. If existing mutations are not close to a catalytic domain, an active site, or another functional domain, it can be expected that there is a small impact.

Those skilled in the art can identify essential amino acids of the Cas protein of the present disclosure according to a method known in the art, such as site-directed mutagenesis or protein evolution or bioinformatics analysis. The catalytic domain, active site, or another functional domain of the protein can also be determined by physical analysis of the structure, for example, it can be determined through a technique such as nuclear magnetic resonance (NMR), crystallography, electron diffraction, or photoaffinity labeling in combination with a putative amino acid mutation at a key position.

The biological function includes, but is not limited to, activity to bind to gRNA, endonuclease activity, and activity to bind to a specific site of a target sequence and cleave the target sequence under guidance of gRNA (including but not limited to Cis cleavage activity and Trans cleavage active).

The present disclosure also provides a fusion protein including the Cas protein described above and other modification moiety.

In an embodiment, the modification moiety is selected from another protein or polypeptide, a detectable label, or any combination thereof.

In an embodiment, the modification moiety is selected from an epitope tag, a reporter gene sequence, a nuclear localization signal (NLS) sequence, a targeting moiety, a transcriptional activation domain (such as VP64), a transcriptional repression domain (such as KRAB or SID domain), a nuclease domain (such as Fok1), and a domain with activity selected from nucleotide deaminase activity, methylase activity, demethylase activity, transcription activation activity, transcription repression activity, transcription release factor activity, histone modification activity, nuclease activity, single-stranded RNA cleavage activity, double-stranded RNA cleavage activity, single-stranded DNA cleavage activity, double-stranded DNA cleavage activity, and nucleic acid binding activity; and any combination thereof. The NLS sequence is well known to those skilled in the art, and examples thereof include, but are not limited to, SV40 large T antigen, EGL-13, c-Myc, and TUS protein.

In an embodiment, the NLS sequence is located at, close to, or proximate to a terminus (such as N-terminus, C-terminus, or both termini) of the Cas protein of the present disclosure.

The epitope tag is well known to those skilled in the art, including but not limited to His, V5, FLAG, HA, Myc, VSV-G, and Trx; and those skilled in the art can select other suitable epitope tags (such as purification, detection, or tracing tag).

The reporter gene sequence is well known to those skilled in the art, and examples thereof include but are not limited to GST, HRP, CAT, GFP, HcRed, DsRed, CFP, YFP, and BFP.

In an embodiment, the fusion protein of the present disclosure includes a domain capable of binding to a DNA molecule or an intracellular molecule, such as maltose-binding protein (MBP), DNA binding domain (DBD) of Lex A, and DBD of GAL4.

In an embodiment, the fusion protein of the present disclosure includes a detectable label, such as a fluorescent dye, such as fluorescein isothiocyanate (FITC) or 4',6-diamidino-2-phenylindole (DAPI).

In an embodiment, the Cas protein of the present disclosure is optionally coupled to, conjugated with, or fused to the modification moiety through a linker.

In an embodiment, the modification moiety is directly linked to the N-terminus or C-terminus of the Cas protein of the present disclosure.

In an embodiment, the modification moiety is linked to the N-terminus or C-terminus of the Cas protein of the present disclosure through a linker. Such linkers are well known in the art, and examples thereof include, but are not limited to, a linker with one or more (such as 1, 2, 3, 4, or 5) amino acids (such as Glu or Ser) or amino acid derivatives (such as Ahx, β-Ala, GABA, or Ava), or a polyethylene glycol (PEG) linker.

A production method of the Cas protein, protein derivative, or fusion protein of the present disclosure is not limited. For example, the Cas protein, protein derivative, or fusion protein can be produced by a genetic engineering method (recombinant technology), or can be produced by a chemical synthesis method.

### Nucleic acid of Cas protein

In another aspect, the present disclosure provides an isolated polynucleotide, including:
(a) a polynucleotide sequence encoding the Cas protein or fusion protein of the present disclosure;
(b) a polynucleotide with a sequence shown in SEQ ID No. 2;
(c) a sequence obtained through substitution, deletion, or addition of one or more bases (such as substitution, deletion, or addition of 1, 2, 3, 4, 5, 6, 7, 8, 9, or 10 bases) based on a sequence shown in SEQ ID No. 2;
(d) a polynucleotide that has a sequence homology ≥ 80% (preferably ≥ 90%, more preferably ≥ 95%, and most preferably ≥ 98%) with the sequence shown in SEQ ID No. 2 and encodes the polypeptide shown in SEQ ID No. 1; or,
(e) a polynucleotide complementary to any one of the polynucleotides described in (a) to (d).

In an embodiment, the nucleotide sequence described in any one of (a) to (e) is codon-optimized for expression in a prokaryotic cell. In an embodiment, the nucleotide sequence described in any one of (a) to (e) is codon-optimized for expression in a eukaryotic cell.

In an embodiment, the polynucleotide is preferably single-stranded or double-stranded.

### gRNA

In another aspect, the present disclosure provides a gRNA including a trans-activating CRISPR RNA (tracrRNA) and a CRISPR RNA (crRNA). The tracrRNA can pair with a pairing region of the crRNA to produce a duplex. The crRNA further includes a region that hybridizes with a target sequence (namely, a targeting sequence for a target nucleic acid).

It is known in the art that a Cas protein requires the guidance of gRNA to target a target sequence and the gRNA includes tracrRNA and crRNA. A 3' terminus of the tracrRNA can pair with a 5' terminus of the crRNA. A 3' terminus of the crRNA includes a region that hybridizes with a target sequence (namely, a targeting sequence).

In an embodiment, the gRNA (from 5' to 3') includes tracrRNA and crRNA, and the crRNA includes a pairing region sequence for the tracrRNA and a targeting sequence.

In an embodiment, the gRNA (from 5' to 3') includes tracrRNA, a pairing region sequence in a crRNA that targets the tracrRNA, and a targeting sequence of the crRNA.

In an embodiment, the gRNA further includes a linker portion between the tracrRNA and the crRNA. Preferably, a sequence of the linker portion is GAAA.

In an embodiment, the gRNA (from 5' to 3') includes the tracrRNA, the linker portion, the pairing region sequence in the crRNA that targets the tracrRNA, and the targeting sequence of the crRNA.

In an embodiment, the gRNA includes a non-targeting sequence and a targeting sequence sequentially from 5' terminus to 3' terminus, and the non-targeting sequence includes a tracrRNA and a pairing region sequence in a crRNA that targets the tracrRNA.

In an embodiment, the gRNA includes a non-targeting sequence and a targeting sequence sequentially from 5' terminus to 3' terminus, and the non-targeting sequence includes a tracrRNA, a linker portion, and a pairing region sequence in a crRNA that targets the tracrRNA sequentially from 5' terminus to 3' terminus.

In an embodiment, the pairing region sequence in the crRNA that targets the tracrRNA is shown in SEQ ID No. 3.

In an embodiment, a sequence of the tracrRNA is shown in SEQ ID No. 4.

In an embodiment, the non-targeting sequence of the gRNA is shown in SEQ ID No. 5, or, the non-targeting sequence of the gRNA includes a base mutation relative to SEQ ID No. 5.

In an embodiment, the base mutation includes base deletion, base substitution, or base insertion.

In an embodiment, the base mutation is selected from any one or more (for example, any 1, 2, 3, 4, or 5) of the following (1) to (20):
(1) deletion of 1st to 12th bases in SEQ ID No. 5;
(2) deletion of 1st to 26th bases in SEQ ID No. 5;
(3) deletion of 13th to 26th and 158th to 172nd bases in SEQ ID No. 5;
(4) substitution of A with C at position 29 and substitution of U with G at position 155 in SEQ ID No. 5;
(5) substitution of U with C at position 31 and substitution of A with G at position 154 in SEQ ID No. 5;
(6) addition of U between U at position 155 and G at position 156 in SEQ ID No. 5;
(7) substitution of A with C at position 29, substitution of U with C at position 31, substitution of A with G at position 154, and substitution of U with G at position 155 in SEQ ID No. 5;
(8) substitution of A with C at position 74 and substitution of U with G at position 88 in SEQ ID No. 5;
(9) substitution of U with C at position 100 and substitution of A with G at position 119 in SEQ ID No. 5;
(10) substitution of U with C at position 105 and substitution of A with G at position 114 in SEQ ID No. 5;
(11) substitution of U with C at position 100, substitution of U with C at position 105, substitution of A with G at position 114, and substitution of A with G at position 119 in SEQ ID No. 5;
(12) substitution of U with G at position 124 and substitution of A with C at position 143 in SEQ ID No. 5;
(13) substitution of A with G at position 126 and substitution of U with C at position 141 in SEQ ID No. 5;
(14) addition of U between G at position 127 and G at position 128 in SEQ ID No. 5;
(15) substitution of U with G at position 124, substitution of A with G at position 126, substitution of U with C at position 141, and substitution of A with C at position 143 in SEQ ID No. 5;
(16) deletion of 198th to 201st bases in SEQ ID No. 5;
(17) deletion of 192nd to 200th bases in SEQ ID No. 5;
(18) deletion of 205th to 209th bases in SEQ ID No. 5;
(19) deletion of 205th to 217th bases in SEQ ID No. 5; and
(20) deletion of 205th to 222nd bases in SEQ ID No. 5.

In an embodiment, the base mutation is selected from any two of the above (1) to (20), for example, (1) and (3), (1) and (13), (3) and (13), (5) and (6), (5) and (13), or (13) and (17).

In an embodiment, the base mutation is a combination of (1) and (3), namely, deletion of 1st to 26th and 158th to 172nd bases.

In an embodiment, the base mutation is a combination of (1) and (13), namely, deletion of 1st to 12th bases, substitution of A with G at position 126, and substitution of U with C at position 141.

In an embodiment, the base mutation is a combination of (3) and (13), namely, deletion of 13th to 26th and 158th to 172nd bases, substitution of A with G at position 126, and substitution of U with C at position 141.

In an embodiment, the base mutation is a combination of (5) and (6), namely, substitution of U with C at position 31, substitution of A with G at position 154, and addition of U between U at position 155 and G at position 156.

In an embodiment, the base mutation is a combination of (5) and (13), namely, substitution of U with C at position 31, substitution of A with G at position 154, substitution of A with G at position 126, and substitution of U with C at position 141.

In an embodiment, the base mutation is a combination of (13) and (17), namely, substitution of A with G at position 126, substitution of U with C at position 141, and deletion of 192nd to 200th bases.

In an embodiment, a sequence of the gRNA includes a sequence shown in any one of SEQ ID Nos. 5-31.

In an embodiment, the non-targeting sequence of the gRNA is shown in any one of SEQ ID Nos. 5-31.

In an embodiment, the gRNA of the present disclosure (also referred to as guide RNA or guiding RNA) includes crRNA and tracrRNA molecules that are partially complementary to produce a complex. The crRNA includes a sequence that exhibits a sufficient complementarity for a target sequence to hybridize with a complementary sequence of the target sequence and to guide a Cas enzyme to bind to the target sequence in a sequence-specific manner. The gRNA of the present disclosure includes tracrRNA and crRNA.

The nucleic acid-targeted targeting sequence or the nucleic acid-targeted targeting segment of the present disclosure includes a nucleotide sequence complementary to a sequence in the target nucleic acid. In other words, the nucleic acid-targeted targeting sequence or the nucleic acid-targeted targeting segment of the present disclosure can interact with the target nucleic acid in a sequence-specific manner through hybridization (namely, base pairing). Therefore, the nucleic acid-targeted targeting sequence or the nucleic acid-targeted targeting segment can be changed, or can be modified to hybridize with any desired sequence in the target nucleic acid. The nucleic acid is selected from DNA and RNA.

The nucleic acid-targeted targeting sequence or the nucleic acid-targeted targeting segment has at least 60% (such as at least 65%, at least 70%, at least 75%, at least 80%, at least 85%, at least 90%, at least 95%, at least 97%, at least 98%, at least 99%, or 100%) complementarity with a target sequence of the target nucleic acid.

The gRNA of the present disclosure guides the Cas protein interacting therewith to a specific nucleotide sequence in the target nucleic acid under the action of the nucleic acid-targeted targeting sequence.

The gRNA of the present disclosure can form a complex with the Cas protein.

The gRNA for the Cas-sf6728 protein of the present disclosure includes a targeting sequence that hybridizes with a target nucleic acid. The target nucleic acid includes a sequence located at a 3' terminus of PAM, and the PAM is 5'-AAN-3', where N represents A/C/G/T.

### Vector

The present disclosure also provides a vector, including the Cas protein, the isolated nucleic acid, or the polynucleotide described above. Preferably, the vector also includes a regulatory element operably linked to the Cas protein, the isolated nucleic acid, or the polynucleotide.

In an embodiment, the regulatory element is one or more selected from the group consisting of an enhancer, a transposon, a promoter, a terminator, a leader sequence, a polyadenylate sequence, and a marker gene.

In an embodiment, the vector includes a cloning vector, an expression vector, a shuttle vector, and an integration vector.

In some embodiments, a vector included in the system is a viral vector (such as a retroviral vector, a lentiviral vector, an adenoviral vector, an adeno-associated virus (AAV) vector, or a herpes simplex virus (HSV) vector), or may also be a plasmid, a virus, a cosmid, or a phage, which are well known to those skilled in the art.

### CRISPR system

The present disclosure provides an engineered non-natural vector system or a CRISPR-Cas system, which includes the Cas protein or a nucleic acid sequence encoding the Cas protein, and a nucleic acid encoding one or more gRNAs. The one or more gRNAs include a region that binds to the Cas protein and a targeting sequence that targets a nucleic acid, or are the gRNA described above.

In an embodiment, the nucleic acid sequence encoding the Cas protein and the nucleic acid encoding one or more gRNAs are artificially synthesized.

In an embodiment, the nucleic acid sequence encoding the Cas protein and the nucleic acid encoding one or more gRNAs do not co-exist naturally.

The one or more gRNAs target one or more target sequences in the cell. The one or more target sequences hybridize with a genomic locus of a DNA molecule encoding one or more gene products, and guide the Cas protein to the genomic locus of the DNA molecule encoding the one or more gene products; and after the Cas protein reaches the position of the target sequence, the target sequence is modified, edited, or cleaved, such that the expression of the one or more gene products is changed or modified.

The cell of the present disclosure includes one or more of an animal cell, a plant cell, or a microorganism.

In some embodiments, the Cas protein is codon-optimized for expression in a cell.

In some embodiments, the Cas protein guides the cleavage of one or two strands at the position of the target sequence.

The present disclosure also provides an engineered non-natural vector system, including one or more vectors; and the one or more vectors include:
a) a first regulatory element operably linked to the gRNA and
b) a second regulatory element operably linked to the Cas protein;
where the components (a) and (b) are located on the same vector or different vectors of the system.

The first and second regulatory elements include a promoter (such as a constitutive promoter or an inducible promoter), an enhancer (such as a 35S promoter or a 35S enhanced promoter), an internal ribosome entry site (IRES), and other expression control elements (such as a transcriptional termination signal, such as a polyadenylation signal and a poly-U sequence).

In some embodiments, a vector in the system is a viral vector (such as a retroviral vector, a lentiviral vector, an adenoviral vector, an AAV vector, or an HSV vector), or may also be a plasmid, a virus, a cosmid, or a phage, which are well known to those skilled in the art.

In some embodiments, the system provided herein is in a delivery system. In some embodiments, the delivery system is a nanoparticle, a liposome, an exosome, a microvesicle, or a gene gun.

In an embodiment, the target sequence is a DNA or RNA sequence derived from a prokaryotic cell or a eukaryotic cell. In an embodiment, the target sequence is a non-natural DNA or RNA sequence.

In an embodiment, the target sequence is present in a cell. In an embodiment, the target sequence is present in the nucleus or cytoplasm (such as an organelle). In an embodiment, the cell is a eukaryotic cell. In other embodiments, the cell is a prokaryotic cell.

In an embodiment, the Cas protein is linked to one or more NLS sequences. In an embodiment, the fusion protein includes one or more NLS sequences. In an embodiment, the NLS sequence is linked to the N-terminus or C-terminus of the protein. In an embodiment, the NLS sequence is fused to the N-terminus or C-terminus of the protein.

In another aspect, the present disclosure relates to an engineered CRISPR system, including the Cas protein and one or more gRNAs. The one or more gRNAs include a region that binds to the Cas protein and a targeting sequence that targets a nucleic acid. The Cas protein can bind to the gRNA and target a target nucleic acid sequence complementary to the targeting sequence. Alternatively, the one or more gRNAs are the gRNA described above.

In an embodiment, the Cas enzyme is Cas-sf6728, and the target nucleic acid is DNA (preferably, double-stranded DNA). The target nucleic acid is located at a 3' terminus of PAM, and the PAM is 5'-AAN-3', where N represents A/C/G/T.

### Protein-nucleic acid complex/composition

In another aspect, the present disclosure provides a complex or a composition, including:
(i) a protein component selected from the Cas protein, the derivatized protein, or the fusion protein and any combination thereof; and
(ii) a nucleic acid component selected from a gRNA or a nucleic acid encoding the gRNA or a precursor RNA of the gRNA or a nucleic acid encoding the precursor RNA of the gRNA, where the gRNA includes a region that binds to the Cas protein and a targeting sequence that targets a nucleic acid.

The protein component can bind to the nucleic acid component to produce a complex.

In an embodiment, the nucleic acid component is a gRNA in the CRISPR-Cas system.

In an embodiment, the complex or composition is non-natural or modified. In an embodiment, at least one component in the complex or composition is non-natural or modified. In an embodiment, the first component is non-natural or modified, and/or, the second component is non-natural or modified.

### Activated CRISPR complex

In another aspect, the present disclosure also provides an activated CRISPR complex, including: (1) a protein component selected from the Cas protein, the derivatized protein, or the fusion protein of the present disclosure and any combination thereof; (2) a nucleic acid component selected from a gRNA or a nucleic acid encoding the gRNA or a precursor RNA of the gRNA or a nucleic acid encoding the precursor RNA of the gRNA, where the gRNA includes a region that binds to the Cas protein and a targeting sequence that targets a nucleic acid; and (3) a target sequence binding to the gRNA. Preferably, the binding refers to the binding to a target nucleic acid through a nucleic acid-targeted targeting sequence of the gRNA.

The term "activated CRISPR complex", "activated complex", or "ternary complex" as used herein refers to a complex obtained after the Cas protein and gRNA in the CRISPR system bind to or are modified by a target nucleic acid.

The Cas protein and gRNA of the present disclosure can form a binary complex that is activated when binding to a nucleic acid substrate to form an activated CRISPR complex, where the nucleic acid substrate is complementary to a targeting sequence (or called a guide sequence to hybridize with the target nucleic acid) in the gRNA. In some embodiments, the targeting sequence of the gRNA may exactly match the target substrate. In other embodiments, the targeting sequence of the gRNA may match a portion (continuous or discontinuous) of the target substrate.

In a preferred embodiment, the activated CRISPR complex may exhibit side-branch nuclease cleavage activity, and the side-branch nuclease cleavage activity refers to non-specific cleavage activity or disordered cleavage activity (which is also called trans cleavage activity in the art) of the activated CRISPR complex on a single-stranded nucleic acid.

### Delivery and delivery composition

The Cas protein, gRNA, fusion protein, nucleic acid, vector, system, complex, and composition of the present disclosure can be delivered by any method known in the art. Such a method includes, but is not limited to, electroporation, lipofection, nucleofection, microinjection, sonoporation, gene gun, calcium phosphate-mediated transfection, cationic lipid transfection, lipofectin transfection, dendritic transfection, heat-shock transfection, nucleofection, magnetofection, lipofection, puncture transfection, optical transfection, reagent-enhanced nucleic acid intake, and delivery via a liposome, an immunoliposome, a viral particle, an artificial virus, etc.

Therefore, in another aspect, the present disclosure provides a delivery composition, which includes a delivery vehicle and selected from one or more of the Cas protein, fusion protein, nucleic acid, vector, system, complex, and composition of the present disclosure.

In an embodiment, the delivery vehicle is a particle.

In an embodiment, the delivery vehicle is selected from a lipid particle, a sugar particle, a metal particle, a protein particle, a liposome, an exosome, a microvesicle, a gene guns, and a viral vector (such as replication-defective retrovirus, lentivirus, adenovirus, or AAV).

### Host cell

The present disclosure also relates to a cell or cell line or progeny thereof *in vitro* or *in vivo,* and the cell or cell line or progeny thereof includes the Cas protein, the fusion protein, the nucleic acid, the gRNA, the protein-nucleic acid complex, the activated CRISPR complex, the vector, or the delivery compositions of the present disclosure.

In some embodiments, the cell is a prokaryotic cell.

In some embodiments, the cell is a eukaryotic cell. In some embodiments, the cell is a mammalian cell. In some embodiments, the cell is a human cell. In some embodiments, the cell is a non-human mammalian cell, such as a cell of a non-human primate, cow, sheep, pig, dog, monkey, rabbit, or a rodent (such as rat or mouse). In some embodiments, the cell is a non-mammalian eukaryotic cell, such as a cell of a poultry bird (such as chicken), fish, or crustacea (such as clam or shrimp). In some embodiments, the cell is a plant cell, such as a cell possessed by a monocotyledonous plant or a dicotyledonous plant or a cell possessed by a cultivated plant or a food crop such as cassava, corn, sorghum, soybean, wheat, oats, or rice. For example, the cell is a cell possessed by algae, a tree, a production plant, a fruit, or a vegetable (for example, a tree such as a citrus tree or a nut tree; or a nightshade, cotton, tobacco, tomato, grape, coffee, cocoa, etc.).

In some embodiments, the cell is a stem cell or a stem cell line.

In some cases, the host cell of the present disclosure includes a gene or a genome modification that is not present in the WT of the host cell.

### Gene editing method and use

The Cas protein, the nucleic acid, the gRNA, the composition, the CIRSPR/Cas system, the vector system, the delivery composition, the activated CRISPR complex, or the host cell of the present disclosure can be used in one or more selected from the group consisting of targeting and/or editing a target nucleic acid; cleaving a double-stranded DNA, a single-stranded DNA, or a single-stranded RNA; non-specifically cleaving and/or degrading a side-branch nucleic acid; non-specifically cleaving a single-stranded nucleic acid; nucleic acid detection; detecting a nucleic acid in a target sample; specifically editing a double-stranded nucleic acid; base-editing a double-stranded nucleic acid; and base-editing a single-stranded nucleic acid. In other embodiments, the products of the present disclosure can also be used to prepare a reagent or a kit for one or more of the above purposes.

The present disclosure also provides a use of the Cas protein, the nucleic acid, the gRNA, the composition, the CIRSPR/Cas system, the vector system, the delivery composition, or the activated CRISPR complex in gene editing, gene targeting, or gene cleaving; or use thereof in the preparation of a reagent or kit for gene editing, gene targeting, or gene cleaving.

In an embodiment, the gene editing, gene targeting, or gene cleaving includes gene editing, gene targeting, or gene cleaving inside and/or outside a cell.

The present disclosure also provides a method for editing, targeting, or cleaving a target nucleic acid, including: contacting the target nucleic acid with the Cas protein, the nucleic acid, the gRNA, the composition, the CIRSPR/Cas system, the vector system, the delivery composition, or the activated CRISPR complex. In an embodiment, the method includes: editing, targeting, or cleaving the target nucleic acid inside or outside a cell.

The gene editing or the editing a target nucleic acid includes modifying a gene, knocking out a gene, changing the expression of a gene product, repairing a mutation, and/or inserting a polynucleotide or a gene mutation.

The editing can be conducted in a prokaryotic cell and/or a eukaryotic cell.

In another aspect, the present disclosure also provides a use of the Cas protein, the nucleic acid, the gRNA, the composition, the CIRSPR/Cas system, the vector system, the delivery composition, or the activated CRISPR complex in nucleic acid detection; or use thereof in the preparation of a reagent or kit for nucleic acid detection.

In another aspect, the present disclosure also provides a method for cleaving a single-stranded nucleic acid, including: contacting a nucleic acid group with the Cas protein and the gRNA described above, where the nucleic acid group includes a target nucleic acid and a plurality of non-target single-stranded nucleic acids, and the Cas protein cleaves the plurality of non-target single-stranded nucleic acids.

The gRNA can bind to the Cas protein.

The gRNA can target the target nucleic acid.

The contacting can be conducted inside a cell *in vitro* or *in vivo.*

Preferably, the cleavage of the single-stranded nucleic acid includes non-specific cleavage.

In another aspect, the present disclosure also provides use of the Cas protein, the nucleic acid, the gRNA, the composition, the CIRSPR/Cas system, the vector system, the delivery composition, or the activated CRISPR complex in the non-specific cleavage of a single-stranded nucleic acid; or use thereof in the preparation of a reagent or kit for the non-specific cleavage of a single-stranded nucleic acid.

In another aspect, the present disclosure also provides a kit for gene editing, gene targeting, or gene cleaving, including the Cas protein, the nucleic acid, the gRNA, the composition, the CIRSPR/Cas system, the vector system, the delivery composition, the activated CRISPR complex, or the host cell.

In another aspect, the present disclosure also provides a kit for detecting a target nucleic acid in a sample, including: (a) the Cas protein or a nucleic acid encoding the Cas protein; (b) the gRNA, or a nucleic acid encoding the gRNA, or a precursor RNA including the gRNA, or a nucleic acid encoding the precursor RNA; and (c) a single-stranded nucleic acid detector that does not hybridize with the gRNA.

It is known in the art that the precursor RNA can be cleaved or processed into the above-mentioned mature gRNA.

In another aspect, the present disclosure provides a use of the Cas protein, the nucleic acid, the gRNA, the composition, the CIRSPR/Cas system, the vector system, the delivery composition, the activated CRISPR complex, or the host cell in the preparation of a formulation or a kit, where the preparation or the kit is used for:
(i) gene or genome editing;
(ii) target nucleic acid detection and/or diagnosis;
(iii) editing a target sequence in a target gene locus to modify an organism or a non-human organism;
(iv) disease treatment; and
(v) targeting a target gene.
(vi) cleaving a target gene

Preferably, the gene or genome editing is conducted inside or outside a cell.

Preferably, the target nucleic acid detection and/or diagnosis includes target nucleic acid detection and/or diagnosis *in vitro.*

Preferably, the disease treatment includes treatment of a disease caused by a defect of a target sequence in a target gene locus.

In another aspect, the present disclosure provides a method for detecting a target nucleic acid in a sample, including: contacting the sample with the Cas protein, a gRNA, and a single-stranded nucleic acid detector; and detecting a detectable signal generated due to cleavage of the Cas protein on the single-stranded nucleic acid detector to detect the target nucleic acid; where the gRNA includes a region that binds to the Cas protein and a targeting sequence that hybridizes with a target nucleic acid, and the single-stranded nucleic acid detector does not hybridize with the gRNA.

### Method for specifically modifying a target nucleic acid

In another aspect, the present disclosure also provides a method for specifically modifying a target nucleic acid, including: contacting the target nucleic acid with the Cas protein, the nucleic acid, the gRNA, the composition, the CIRSPR/Cas system, the vector system, the delivery composition, or the activated CRISPR complex.

This specific modification may occur *in vivo* or *in vitro.*

This specific modification may occur inside or outside a cell.

In some cases, the cell is selected from a prokaryotic cell or a eukaryotic cell, such as an animal cell, a plant cell, or a microbial cell.

In an embodiment, the modification includes a break in the target sequence, such as a single-strand break (SSB)/DSB in DNA or an SSB in RNA.

In some cases, the method further includes contacting the target nucleic acid with a donor polynucleotide, where the donor polynucleotide, a portion of the donor polynucleotide, a copy of the donor polynucleotide, or a portion of the copy of the donor polynucleotide is integrated into the target nucleic acid.

In an embodiment, the modification further includes inserting an edit template (such as an exogenous nucleic acid) into the break.

In an embodiment, the method further includes contacting an edit template with the target nucleic acid or delivering the edit template to a cell with the target nucleic acid. In an embodiment, the method repairs the broken target gene through homologous recombination with an exogenous template polynucleotide. In some embodiments, the repair results in a mutation, including insertion, deletion, or substitution of one or more nucleotides in the target gene. In other embodiments, the mutation results in one or more amino acid changes in a protein expressed by a gene carrying the target sequence.

### Detection (non-specific cleavage)

In another aspect, the present disclosure provides a method for detecting a target nucleic acid in a sample, including: contacting the sample with the Cas protein, the nucleic acid, the gRNA, the composition, the CIRSPR/Cas system, the vector system, the delivery composition, or the activated CRISPR complex and the single-stranded nucleic acid detector; and detecting a detectable signal generated due to cleavage of the Cas protein on the single-stranded nucleic acid detector to detect the target nucleic acid.

In the present disclosure, the target nucleic acid includes ribonucleotides or deoxyribonucleotides; and the target nucleic acid includes a single-stranded nucleic acid and a double-stranded nucleic acid, such as single-stranded DNA, double-stranded DNA, single-stranded RNA, and double-stranded RNA.

In an embodiment, the target nucleic acid is derived from a sample such as a virus, a bacterium, a microorganism, soil, a water source, a human body, an animal, and a plant. Preferably, the target nucleic acid is a product of enrichment or amplification by a method such as PCR, NASBA, RPA, SDA, LAMP, HAD, NEAR, MDA, RCA, LCR, or RAM.

In an embodiment, the target nucleic acid is a viral nucleic acid, a bacterial nucleic acid, or a disease-specific nucleic acid, such as a specific mutation site or SNP site or a nucleic acid that differs from a control. Preferably, the virus is a plant virus or an animal virus, for example, papillomavirus, hepadnavirus, herpesvirus, adenovirus, poxvirus, parvovirus, and coronavirus. Preferably, the virus is a coronavirus, including SARS, SARS-CoV2 (COVID-19), HCoV-229E, HCoV-OC43, HCoV-NL63, HCoV-HKU1, and Mers-CoV.

In the present disclosure, the gRNA and the target sequence on the target nucleic acid have a matching degree of at least 50%, preferably at least 60%, preferably at least 70%, preferably at least 80%, and preferably at least 90%.

In an embodiment, when the target sequence includes one or more characteristic sites (such as specific mutation sites or SNPs), the characteristic sites completely match the gRNA.

In an embodiment, the detection method includes one or more gRNAs with different guide sequences, which target different target sequences.

In the present disclosure, the single-stranded nucleic acid detector includes, but is not limited to, a single-stranded DNA, a single-stranded RNA, a DNA-RNA hybrid, a nucleic acid analogue, a base modifier, and a single-stranded nucleic acid detector with an abasic spacer; and the nucleic acid analogue includes, but is not limited to, locked nucleic acid (LNA), bridged nucleic acid (BNA), morpholino, glycol nucleic acid (GNA), hexitol nucleic acid (HNA), threose nucleic acid (TNA), arabinose nucleic acid (ANA), 2'-O-methyl RNA, 2'-methoxyacetyl RNA, 2'-fluoro RNA, 2'-amino RNA, 4'-thio RNA, and a combination thereof, including optional ribonucleotide or deoxyribonucleotide residues.

In the present disclosure, the detectable signal is detected in the following ways: vision-based detection, sensor-based detection, color detection, fluorescence signal-based detection, gold nanoparticle-based detection, fluorescence polarization, colloidal phase change/dispersion, electrochemical detection, and semiconductor-based detection.

In the present disclosure, preferably, two termini of the single-stranded nucleic acid detector is provided with a fluorophore and a quencher respectively; and when the single-stranded nucleic acid detector is cleaved, a detectable fluorescence signal can be presented. The fluorophore is one or more from the group consisting of FAM, FITC, VIC, JOE, TET, CY3, CY5, ROX, Texas Red, and LC RED460; and the quencher is one or more from the group consisting of BHQ1, BHQ2, BHQ3, Dabcyl, and Tamra.

In other embodiments, a 5' terminus and a 3' terminus of the single-stranded nucleic acid detector is provided with different labeling molecules respectively. The single-stranded nucleic acid detector is subjected to a colloidal gold test before and after being cleaved by the Cas protein; and the single-stranded nucleic acid detector shows different chromogenic results on the colloidal gold detection line and control line before and after being cleaved by the Cas protein.

In some embodiments, the method for detecting a target nucleic acid further includes: comparing a level of the detectable signal with a reference signal level, and determining an amount of the target nucleic acid in the sample based on the level of the detectable signal.

In some embodiments, the method for detecting a target nucleic acid may also include: using a RNA reporter nucleic acid and a DNA reporter nucleic acid (such as fluorescence color) on different channels, measuring a signal level of the RNA and DNA reporter molecules and an amount of the target nucleic acid in the RNA and DNA reporters to determine a level of the detectable signal, and sampling based on a combined (such as minimum or product) level of the detectable signal.

In an embodiment, the target gene is present in a cell.

In an embodiment, the cell is a prokaryotic cell.

In an embodiment, the cell is a eukaryotic cell.

In an embodiment, the cell is an animal cell.

In an embodiment, the cell is a human cell.

In an embodiment, the cell is a plant cell, such as a cell possessed by a cultivated plant (such as cassava, corn, sorghum, wheat, or rice), algae, a tree, or a vegetable.

In an embodiment, the target gene is present in a nucleic acid *in vitro* (such as a plasmid).

In an embodiment, the target gene is present in a plasmid.

### Terms and definitions

In the present disclosure, unless otherwise specified, the scientific and technical terms used herein have the meanings commonly understood by those skilled in the art. In addition, the molecular genetics, nucleic acid chemistry, chemistry, molecular biology, biochemistry, cell culture, microbiology, cell biology, genomics, and recombinant DNA operation procedures used herein are routine procedures widely used in the corresponding fields. Moreover, in order to better explain the present disclosure, definitions and explanations of related terms are provided below.

In the present disclosure, an amino acid residue can be represented by a single letter or three letters, such as: alanine (Ala, A), valine (Val, V), glycine (Gly, G), leucine (Leu, L), glutamine (Gln, Q), phenylalanine (Phe, F), tryptophan (Trp, W), tyrosine (Tyr, Y), aspartic acid (Asp, D), asparagine (Asn, N), glutamic acid (Glu, E), lysine (Lys, K), methionine (Met, M), serine (Ser, S), threonine (Thr, T), cysteine (Cys, C), proline (Pro, P), isoleucine (Ile, I), histidine (His, H), and arginine (Arg, R).

The term "AxxB" means that an amino acid A at position xx is mutated to an amino acid B, that is, the amino acid A at the position xx from an N-terminus is mutated to the amino acid B, unless otherwise specified. For example, L65R indicates that L at position 65 is mutated to R. When there are mutations simultaneously at a plurality of amino acid sites, the mutations can be expressed in a form such as L65R-V75R, L65R V75R, or L65R/V75R. For example, L65R-V75R means that L at position 65 is mutated to R and V at position 75 is mutated to R.

A specific amino acid position (number) in the protein of the present disclosure is determined by aligning an amino acid sequence of the target protein with SEQ ID No. 1 using a standard sequence alignment tool. For example, the Smith-Waterman algorithm or the CLUSTALW2 algorithm is used to align two sequences, and the sequences are considered aligned when an alignment score is the highest. The alignment score can be calculated according to the method described in Wilbur, W. J. and Lipman, D. J. (1983) Rapid similarity searches of nucleic acid and protein data banks. Proc. Natl. Acad. Sci. USA, 80: 726-730. In the ClustalW2(1.82) algorithm, default parameters are preferably adopted: protein gap opening penalty = 10.0; protein gap extension penalty = 0.2; protein matrix = Gonnet; protein/DNA end gap = -1; and protein/DNAGAPDIST = 4. The AlignX program (a part of the vectorNTI group) may preferably be used to align an amino acid sequence of the protein of the present disclosure with SEQ ID No. 1 under default parameters suitable for multiple alignment (gap opening penalty: 10; and gap extension penalty: 0.05) to determine a position of a specific amino acid in the protein.

Those skilled in the art can use the common software in the art, such as Clustal Omega, to compare and align an amino acid sequence of any parental Cas protein with SEQ ID No. 1 for sequence identity, so as to obtain an amino acid site in the parental Cas protein that corresponds to an amino acid site defined based on SEQ ID No. 1 in the present application.

### Cas protein

In the present disclosure, the terms "Cas protein", "Cas enzyme", and "Cas effector protein" can be used interchangeably. The inventors have discovered and identified a Cas effector protein for the first time, which has an amino acid sequence selected from the following sequences:
(i) a sequence possessing a sequence identity of at least 20%, at least 30%, at least 40%, at least 50%, at least 60%, at least 70%, at least 80%, at least 80%, at least 85%, at least 90%, at least 91%, at least 92%, at least 93%, at least 94%, at least 95%, at least 96%, at least 97%, at least 98%, or at least 99% with a sequence shown in SEQ ID No. 1;
(ii) a sequence including substitution, deletion, or addition of one or more amino acids (such as substitution, deletion, or addition of 1, 2, 3, 4, 5, 6, 7, 8, 9, or 10 amino acids) relative to the sequence shown in SEQ ID No. 1; and
(iii) a sequence that has a sequence identity of at least 80% with the sequence shown in SEQ ID No. 1, and includes a mutation of any one or more (for example, any 1, 2, 3, 4, or 5) of 36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, and 344th amino acids relative to a sequence of a parental Cas protein corresponding to the sequence shown in SEQ ID No. 1.

The nucleic acid cleavage or the cleavage of a nucleic acid herein includes: DNA or RNA break in a target nucleic acid caused by the Cas enzyme described herein (Cis cleavage), and DNA or RNA break in a side-branch nucleic acid substrate (single-stranded nucleic acid substrate) (namely, non-specific or non-targeting Trans cleavage). In some embodiments, the cleavage includes a double-stranded DNA break. In some embodiments, the cleavage includes a single-stranded DNA break or a single-stranded RNA break.

### CRISPR system

The terms "CRISPR-Cas system" and "CRISPR system" used herein can be used interchangeably and have the meaning commonly understood by those skilled in the art, which usually includes a transcription product or other elements related to the expression of a Cas gene, or a transcription product or other elements capable of guiding the activity of the Cas gene.

### CRISPR/Cas complex

As used herein, the term "CRISPR/Cas complex" refers to a complex formed by the binding of a gRNA or mature crRNA to the Cas protein. The complex can recognize and cleave a polynucleotide capable of hybridizing with the gRNA or mature crRNA.

### gRNA

As used herein, the terms "gRNA", "mature crRNA", and "guide sequence" can be used interchangeably and have the meanings commonly understood by those skilled in the art. Generally, gRNA includes tracrRNA and crRNA. The tracrRNA can pair with a pairing region of the crRNA to produce a duplex. The crRNA further includes a region that hybridizes with a target sequence (namely, a targeting sequence for a target nucleic acid). The crRNA includes a pairing region sequence capable of pairing with the tracrRNA, and further includes a region that hybridizes with a target sequence (namely, a targeting sequence for a target nucleic acid).

In some cases, the targeting sequence can be any polynucleotide sequence that shows sufficient complementarity with a target sequence to hybridize with the target sequence and guide the specific binding of the CRISPR/Cas complex to the target sequence. In an embodiment, under optimal alignment, a complementarity degree between the targeting sequence and a corresponding target sequence is at least 50%, at least 60%, at least 70%, at least 80%, at least 90%, at least 95%, or at least 99%. Determining the optimal alignment is within the competence of those of ordinary skill in the art. For example, there are published and commercially available alignment algorithms and programs, including but not limited to Smith-Waterman, Bowtie, Geneious, Biopython, and SeqMan in ClustalW and matlab.

### Target sequence

"Target sequence" refers to a polynucleotide targeted by a targeting sequence in gRNA, such as a sequence that has complementarity with the targeting sequence, where the hybridization between the target sequence and the targeting sequence will promote the formation of a CRISPR/Cas complex (including Cas protein and gRNA). Complete complementarity is not necessary, as long as there is sufficient complementarity to cause hybridization and promote the formation of a CRISPR/Cas complex.

The target sequence can include any polynucleotide, such as DNA or RNA. In some cases, the target sequence is located inside or outside a cell. In some cases, the target sequence is located in the nucleus or cytoplasm of a cell. In some cases, the target sequence is located in an organelle of a eukaryotic cell such as a mitochondrion or a chloroplast. A sequence or a template that can be recombined into a target gene locus with the target sequence is called "edit template", "edit polynucleotide", or "edit sequence". In an embodiment, the edit template is an exogenous nucleic acid. In an embodiment, the recombination includes homologous recombination.

In the present disclosure, the "target sequence", "target polynucleotide", or "target nucleic acid" can be any endogenous or exogenous polynucleotide for a cell (such as a eukaryotic cell). For example, the target polynucleotide is a polynucleotide present in the nucleus of a eukaryotic cell. The target polynucleotide is a sequence encoding a gene product (such as a protein) or a non-coding sequence (such as a regulatory polynucleotide or useless DNA). In some cases, the target sequence should be related to PAM.

### Single-stranded nucleic acid detector

The single-stranded nucleic acid detector of the present disclosure refers to a sequence that includes 2 to 200 nucleotides, preferably 2 to 150 nucleotides, preferably 3 to 100 nucleotides, preferably 3 to 30 nucleotides, preferably 4 to 20 nucleotides, and preferably 5 to 15 nucleotides. Preferably, the single-stranded nucleic acid detector is a single-stranded DNA molecule, a single-stranded RNA molecule, or a single-stranded DNA-RNA hybrid.

Two termini of the single-stranded nucleic acid detector include different reporter groups or labeling molecules. When the single-stranded nucleic acid detector is in an initial state (that is, when the single-stranded nucleic acid detector is not cleaved), no reporter signal is presented; and when the single-stranded nucleic acid detector is cleaved, a detectable signal is presented, indicating a detectable difference before and after cleavage.

In an embodiment, the reporter groups or labeling molecules includes fluorophores and quenchers. The fluorophores are one or more selected from FAM, FITC, VIC, JOE, TET, CY3, CY5, ROX, Texas Red, or LC RED460; and the quenchers are one or more selected from BHQ1, BHQ2, BHQ3, Dabcyl, or Tamra.

In an embodiment, the single-stranded nucleic acid detector may have a first molecule (such as FAM or FITC) linked to the 5' terminus and a second molecule (such as biotin) linked to the 3' terminus. The reaction system with a single-stranded nucleic acid detector is used in combination with a flow strip to detect a target nucleic acid (preferably, colloidal gold detection). The flow strip is designed to have two capture lines, where an antibody to bind to a first molecule (namely, an anti-first molecule antibody) is arranged at a sample contact end (colloidal gold), an antibody to bind to the anti-first molecule antibody is arranged at a first line (control line), and an antibody to bind to a second molecule (namely, an anti-second molecule antibody, such as avidin) is arranged at a second line (test line). As a reaction proceeds along the strip, the anti-first molecule antibody binds to the first molecule and carries a cleaved or uncleaved oligonucleotide to the capture line, where a cleaved reporter will bind to the antibody binding to the anti-first molecule antibody at the first capture line; and an uncleaved reporter will bind to the anti-second molecule antibody at the second capture line. The binding of the reporter group to each line will result in a strong readout/signal (such as color). As more reporters are cut, more signals will accumulate at the first capture line, and fewer signals will appear at the second line. In some aspects, the present disclosure relates to use of the flow strip as described herein in the detection of a nucleic acid. In some aspects, the present disclosure relates to a method for detecting a nucleic acid using a flow strip as defined herein, such as a (lateral) flow test or a (lateral) flow immunochromatographic assay. In some aspects, the molecules in the single-stranded nucleic acid detector can be used instead of each other, or positions of the molecules can be changed. As long as a reporting principle is the same as or similar to that of the present disclosure, an improved method is also included in the present disclosure.

The detection method of the present disclosure can be used for quantitative detection of a target nucleic acid to be detected. The quantitative detection index can be quantified according to a signal intensity of a reporter group, for example, according to a luminous intensity of a fluorophore or according to a width of a chromogenic band.

### Wild-type

As used herein, the term "wild-type" has the meaning commonly understood by those skilled in the art, and indicates the typical form of an organism, a strain, or a gene, or a characteristic to distinguish the organism, strain, or gene in nature from a mutant or variant form thereof, which can be isolated from a natural source and is not artificially modified intentionally.

### Derivatization

As used herein, the term "derivatization" refers to the chemical modification to an amino acid, a polypeptide, or a protein, where one or more substituents have been covalently linked to the amino acid, the polypeptide, or the protein. The substituents can also be referred to as side chains.

A derivatized protein is a derivative of a protein. Generally, the derivatization of a protein will not adversely affect the desired activity of the protein (for example, activity to bind to gRNA, endonuclease activity, and activity to bind to a specific site of a target sequence and cleave the target sequence under the guidance of gRNA). That is, a derivative of a protein has the same activity as the protein.

### Derivatized protein

A derivatized protein, also known as "protein derivative", refers to a modified form of a protein, for example, one or more amino acids of the protein can be deleted, inserted, modified, and/or substituted.

### Non-natural

As used herein, the terms "non-natural" and "engineered" can be used interchangeably and refer to human intervention. When these terms are used to describe a nucleic acid or a polypeptide, it means that the nucleic acid or polypeptide is at least substantially isolated from a natural source or separated from at least another component binding to the nucleic acid or polypeptide in nature.

### Orthologue, ortholog

As used herein, the term "orthologue" or "ortholog" has the meaning commonly understood by those skilled in the art. As a further guide, an orthologue of a protein described herein refers to a protein of a different species, which implements the same function as or the similar function to the protein.

### Identity

As used herein, the term "identity" refers to the sequence matching between two polypeptides or between two nucleic acids. When specified positions in two sequences to be compared are occupied by the same base or amino acid monomer subunit (for example, a specified position in each of two DNA molecules is occupied by adenine, or a specified position in each of two peptides is occupied by lysine), the molecules are identical at the position. The "percentage identity" between two sequences is a function of the number of matched positions shared by the two sequences/the number of compared positions × 100. For example, if 6 of 10 positions in a sequence match corresponding positions in another sequence, then the two sequences have 60% identity. For example, DNA sequences CTGACT and CAGGTT have 50% identity (3 of 6 positions are matching). Generally, the comparison is conducted when two sequences are aligned to produce maximum identity. Such alignment can be achieved by using, for example, a method of Needleman et al. (1970) J. Mol. Biol. 48: 443-453 that can be conveniently implemented by a computer program such as Align program (DNAstar, Inc.). The percentage identity between two amino acid sequences can also be determined by using an algorithm of E. Meyers and W. Miller (Comput. Appl Biosci., 4:11-17 (1988) integrated into the ALIGN program (version 2.0), a PAM120 weight residue table, a gap length penalty of 12, and a gap length penalty of 4. In addition, the percentage identity between two amino acid sequences can be determined by using Needleman and Wunsch (J MoI Biol. 48: 444-453 (1970)) algorithms in the GAP program integrated into the GCG software package (available on www.gcg.com), a Blossum 62 matrix or a PAM250 matrix, a gap weight of 16, 14, 12, 10, 8, 6, or 4, and a length weight of 1, 2, 3, 4, 5, or 6.

### Vector

The term "vector" refers to a nucleic acid molecule that can deliver another nucleic acid linked thereto. The vector includes, but is not limited to, a single-stranded, double-stranded, or partially double-stranded nucleic acid; a nucleic acid with one or more free ends or without free ends (such as circular); DNA, RNA, or a nucleic acid of both; and other diverse polynucleotides known in the art. The vector can be introduced into a host cell through transformation, transduction, or transfection, such that a genetic material element carried by the vector can be expressed in the host cell. A vector can be introduced into a host cell to produce a transcript, a protein, or a peptide, including the protein, the fusion protein, the isolated nucleic acid, and the like (for example, a CRISPR transcript, such as a nucleic acid transcript, a protein, or an enzyme) described herein. A vector may include a variety of elements to control the expression, including but not limited to a promoter sequence, a transcription initiation sequence, an enhancer sequence, a selection element, and a reporter gene. In addition, the vector may also include a replication origin.

One type of vector is plasmid, which refers to a circular double-stranded DNA loop where an additional DNA fragment can be inserted, for example, by a standard molecular cloning technique.

Another type of vector is a viral vector, in which a virus-derived DNA or RNA sequence is present in a vector for packaging a virus (such as retrovirus, replication-defective retrovirus, adenovirus, replication-defective adenovirus, and AAV). A viral vector also includes a polynucleotide carried by a virus to be transfected into a host cell. Some vectors (for example, bacterial vectors with a bacterial replication origin and episomal mammalian vectors) can autonomously replicate in a host cell into which they are introduced.

Other vectors (such as non-episomal mammalian vectors) will be integrated into a genome of a host cell and thus replicate with the genome of the host cell after being introduced into the host cell. Moreover, some vectors can guide the expression of genes operably linked thereto. Such vectors are referred to as expression vectors herein.

### Host cell

As used herein, the term "host cell" refers to a cell that can be introduced with a vector, including, but not limited to, a prokaryotic cell such as *Escherichia coli* (*E. coli*) or *Bacillus subtilis* (*B. subtilis*), and a eukaryotic cell such as a microbial cell, a fungal cell, an animal cell, and a plant cell.

Those skilled in the art will understand that the design of an expression vector may depend on factors such as the selection of a host cell to be transformed, and a desired expression level.

### Regulatory element

As used herein, the term "regulatory element" is intended to include a promoter, an enhancer, an IRES, and other expression control elements (for example, a transcriptional termination signal, such as a polyadenylation signal and a poly-U sequence), and the detailed description can be seen in Goeddel, "GENE EXPRESSION TECHNOLOGY:METHODS IN ENZYMOLOGY" 185, Academic Press, San Diego, California (1990). In some cases, the regulatory element includes sequences that guide the constitutive expression of a nucleotide sequence in many types of host cells and sequences that guide the expression of the nucleotide sequence only in some host cells (such as a tissue-specific regulatory sequence). A tissue-specific promoter can mainly guide the expression in a desired tissue of interest, such as muscles, neurons, bones, skin, blood, specific organs (such as liver and pancreas), or specific cell types (such as lymphocytes). In some cases, a regulatory element can also guide the expression in a time-dependent manner (such as in a cell cycle-dependent or developmental stage-dependent manner), which may be or may not be tissue or cell type-specific. In some cases, the term "regulatory element" covers enhancer elements, such as WPRE; CMV enhancer; R-U5' fragment in LTR of HTLV-I ((Mol.Cell.Biol., Vol 8 (1): 466-472, 1988); SV40 enhancer; and an intron sequence between exons 2 and 3 of rabbit β-globin (Proc. Natl. Acad. Sci. USA., Vol. 78 (3): 1527-31, 1981).

### Promoter

As used herein, the term "promoter" has the meaning well known to those skilled in the art, which refers to a non-coding nucleotide sequence located upstream of a gene and capable of promoting the expression of a downstream gene. A constitutive promoter is a nucleotide sequence that will result in the generation of a gene product in a cell under most or all physiological conditions of the cell after the promoter is operably linked to a polynucleotide encoding or defining the gene product. An inducible promoter is a nucleotide sequence that will cause the generation of a gene product in a cell only when there is an inducer corresponding to the promoter in the cell after the promoter is operably linked to a polynucleotide encoding or defining the gene product. A tissue-specific promoter is a nucleotide sequence that will cause the generation of a gene product in a cell basically only when the cell is a cell of the tissue type corresponding to the promoter after the promoter is operably linked to a polynucleotide encoding or defining a gene product.

### NLS

"NLS" is an amino acid sequence that tags a protein for import into the nucleus through nuclear transport, that is, a protein with NLS is transported to the nucleus. Typically, NLS includes positively charged Lys or Arg residues that are exposed on the surface of a protein. Exemplary NLS includes, but is not limited to, SV40 large T antigen, EGL-13, c-Myc, and TUS protein. In some embodiments, the NLS includes a PKKKRKV sequence. In some embodiments, the NLS includes an AVKRPAATKKAGQAKKKKLD sequence. In some embodiments, the NLS includes a PAAKRVKLD sequence. In some embodiments, the NLS includes an MSRRRKANPTKLSENAKKLAKEVEN sequence. In some embodiments, the NLS includes a KLKIKRPVK sequence. Other NLS includes, but is not limited to, an acidic M9 domain of hnRNP A1, and sequences KIPIK and PY-NLS in the yeast transcription repressor Matα2.

### Operably linked

As used herein, the term "operably linked" means that a nucleotide sequence of interest is linked to one or more regulatory elements in a manner that allows the expression of the nucleotide sequence (for example, in an *in vitro* transcription/translation system or in a host cell when the vector is introduced into the host cell).

### Complementarity

As used herein, the term "complementarity" refers to the ability of a nucleic acid to form one or more hydrogen bonds with another nucleic acid by means of traditional Watson-Crick or another non-traditional form. The complementarity percentage refers to a percentage of residues in a first nucleic acid that can form hydrogen bonds (such as Watson-Crick base pairing) with a second nucleic acid (such as 5, 6, 7, 8, 9, and 10 of 10, namely 50%, 60%, 70%, 80%, 90%, and 100% complementarity). "Completely complementary" means that all consecutive residues of a first nucleic acid sequence form hydrogen bonds with the same number of consecutive residues in a second nucleic acid sequence. As used herein, "substantially complementary" means that there is at least 60%, 65%, 70%, 75%, 80%, 85%, 90%, 95%, 97%, 98%, 99%, or 100% complementarity in a region with 8, 9, 10, 11, 12, 13, 14, 15, 16, 17, 18, 19, 20, 21, 22, 23, 24, 25, 30, 35, 40, 45, 50, or more nucleotides, or means that two nucleic acids can hybridize under stringent conditions.

### Stringent conditions

As used herein, "stringent conditions" for hybridization refer to conditions under which a nucleic acid showing complementarity with a target sequence mainly hybridizes with the target sequence and substantially does not hybridize with a non-target sequence. Stringent conditions are usually sequence-dependent and vary depending on many factors. Generally, the longer the sequence, the higher the temperature at which the sequence specifically hybridizes with a corresponding target sequence.

### Hybridization

The term "hybridization" or "complementary" or "substantially complementary" means that a nucleic acid (such as RNA and DNA) includes a nucleotide sequence that enables its non-covalent binding, that is, the nucleic acid can form base pairs and/or G/U base pairs with another nucleic acid in a sequence-specific, anti-parallel manner (namely, the nucleic acid specifically binds to a complementary nucleic acid), "annealing" or "hybridizing".

The hybridization requires that two nucleic acids include complementary sequences. There are mismatches between bases. Suitable conditions for hybridization between two nucleic acids depend on the length and complementarity degree of the nucleic acids, which are variables well known in the art. Typically, a hybridizable nucleic acid includes 8 nucleotides or more (such as 10 nucleotides or more, 12 nucleotides or more, 15 nucleotides or more, 20 nucleotides or more, 22 nucleotides or more, 25 nucleotides or more, or 30 nucleotides or more).

It should be understood that a sequence of a polynucleotide does not need to be 100% complementary to a sequence of its target nucleic acid for specific hybridization. A polynucleotide may have 60% or more, 65% or more, 70% or more, 75% or more, 80% or more, 85% or more, 90% or more, 95% or more, 98% or more, 99% or more, 99.5% or more, or 100% complementarity with a sequence of a target region in a target nucleic acid sequence to hybridize with the polynucleotide.

The hybridization of the target sequence with the gRNA means that the target sequence and the nucleic acid sequence of gRNA have at least 60%, 70%, 75%, 80%, 85%, 90%, 91%, 92%, 93%, 94%, 95%, 96%, 97%, 98%, 99%, or 100% complementarity, and thus can be hybridized to form a complex; or means that at least 12, 15, 16, 17, 18, 19, 20, 21, 22, or more bases in the target sequence are complementary to and paired with that in the nucleic acid sequence of gRNA, and thus the two sequences can be hybridized to form a complex.

### Expression

As used herein, the term "expression" refers to a process by which a DNA template is transcribed into a polynucleotide (such as mRNA or another RNA transcript) and/or a process by which the transcribed mRNA is subsequently translated into a peptide, a polypeptide, or a protein. The transcript and the encoded polypeptide can be collectively referred to as "gene product". If a polynucleotide is derived from genomic DNA (gDNA), the expression can include splicing of mRNA in a eukaryotic cell.

### Linker

As used herein, the term "linker" refers to a linear polypeptide formed by linking a plurality of amino acid residues through peptide bonds. The linker of the present disclosure may be an artificially-synthesized amino acid sequence, or a natural polypeptide sequence, such as a polypeptide with a hinge domain function. Such linker polypeptides are well known in the art (see, for example, Holliger, P. et al. (1993) Proc. Natl. Acad. Sci. USA 90: 6444-6448; and Poljak, R. J. et al. (1994) Structure 2: 1121-1123).

### Treatment

As used herein, the term "treatment" refers to treating or curing a disease, delaying the onset of symptoms of a disease, and/or delaying the development of a disease.

### Subject

As used herein, the term "subject" includes, but is not limited to, various animals, plants, and microorganisms.

### Animal

For example, the animal may be a mammal, such as bovine, equine, sheep, swine, canine, feline, leporid, rodent (such as mouse or rat), non-human primate (such as macaque or cynomolgus monkey), or human. In some embodiments, the subject (such as human) suffers from a disorder (such as a disorder caused by a disease-related gene defect).

### Plant

The term "plant" should be understood as any differentiated multicellular organism capable of photosynthesis, including: crop plants at a mature or developmental stage, especially monocotyledonous or dicotyledonous plants; vegetable crops including artichoke, turnip cabbage, arugula, leek, asparagus, lettuce (such as head lettuce, leaf lettuce, and romaine lettuce), bok choy, malanga, melons (such as cantaloupe, watermelon, crenshaw melon, honeydew melon, and Roman cantaloupe), rape crops (such as Brussels sprout, cabbage, cauliflower, broccoli, borecole, kale, Chinese cabbage, and bok choy), cardoon, carrot, napa, okra, onion, celery, parsley, chickpea, parsnip, chicory, pepper, potato, gourd (such as marrow squash, cucumber, zucchini, cushaw, and pumpkin), radish, dried ball onion, rutabaga, purple eggplant (also known as eggplant), salsify, lettuce, shallot, endive, garlic, spinach, green onion, cushaw, greens, beets (sugar beets and fodder beets), sweet potato, Swiss chard, wasabi, tomato, turnip, and spices; fruits and/or vine crops such as apple, apricot, cherry, nectarine, peach, pear, plum, prune, cherry, quince, almond, chestnut, hazelnut, pecan, pistachio, walnut, citrus, blueberry, boysenberry, cranberry, currant, loganberry, raspberry, strawberry, blackberry, grape, avocado, banana, kiwi, persimmon, pomegranate, pineapple, tropical fruit, pome, melon, mango, papaya, and lychee; field crops, such as clover, alfalfa, evening primrose, meadowfoam, corn/maize (forage corn, sweet corn, and popcorn), lupulus, jojoba, peanut, rice, safflower, small grain crops (barley, oat, rye, wheat, and the like), sorghum, tobacco, kapok, legumes (beans, lentil, pea, and soybean), oil plants (canola, leaf mustard, olive, sunflower, coconut, castor oil plant, cocoa bean, and groundnut), *Arabidopsis,* fiber plants (cotton, flax, and jute), *Lauraceae* (cinnamon or camphor), or a plant such as coffee, sugar cane, tea, and natural rubber plants; and/or bedding plants such as a flowering plant, cactus, a succulent plant, and/or an ornamental plant, and trees such as forests (broad-leaved and evergreen trees, such as conifers), fruit trees, ornamental trees, nut-bearing trees, shrubs, and other seedlings.

### Advantageous effects of the present disclosure

The present disclosure has discovered a novel Cas enzyme. According to blast results, the Cas enzyme of the present application exhibits a low identity with the Cas enzymes previously reported, indicating that the Cas enzyme of the present application is a novel Cas protein. In the present disclosure, the Cas protein is further mutated to produce Cas mutant proteins with enhanced activities. Thus, the present disclosure has promising application prospects.

Embodiments of the present disclosure will be described in detail below with reference to accompanying drawings and implementations, but those skilled in the art will understand that the following accompanying drawings and examples are used to merely illustrate the present disclosure rather than limit the scope of the present disclosure. Through the following detailed description of accompanying drawings and preferred embodiments, various objects and advantageous aspects of the present disclosure will become apparent to those skilled in the art.

### DESCRIPTION OF THE DRAWINGS

FIG. 1 shows a PAM structure for Cas-sf6728;
FIG. 2 shows editing efficiencies of different single-mutant Cas-sf6728 proteins;
FIG. 3 shows a structure of a non-targeting sequence portion of gRNA for the Cas-sf6728 protein; and
FIG. 4 shows editing efficiencies of different editing systems.

### SPECIFIC IMPLEMENTATIONS

The following examples are only used to describe rather than limit the present disclosure. Unless otherwise specified, the experiments and methods described in the examples are basically conducted in accordance with conventional methods well known in the art and described in various references. For example, conventional techniques such as immunology, biochemistry, chemistry, molecular biology, microbiology, cell biology, genomics, and recombinant DNA used in the present disclosure can be found in *"*MOLECULAR CLONING: A LABORATORY MANUAL", Sambrook, Fritsch, and Maniatis, edition 2 (1989); *"*CURRENT PROTOCOLS IN MOLECULAR BIOLOGY" (edited by F. M. Ausubel et al., (1987)); and *"*METHODS IN ENZYMOLOGY" series (Academic Press Corporation): "PCR 2: A PRACTICAL APPROACH (edited by M. J. MacPherson, B. D. Hames, and G. R. Taylor (1995)), ANTIBODIES, A LABORATORY MANUAL edited by Harlow and Lane (1988), and *"*ANIMAL CELL CULTURE" (edited by R. I. Freshney (1987)).

In addition, if no specific conditions are specified in the examples, the examples will be conducted according to conventional conditions or the conditions recommended by the manufacturer. All of the used reagents or instruments which are not specified with manufacturers are conventional commercially-available products. Those skilled in the art know that the present disclosure is described by way of examples in the embodiments, and the examples are not intended to limit the protection scope of the present disclosure. All publications and other references mentioned herein are incorporated into this article by reference in their entirety.

### Example 1 Acquisition of a Cas-sf6728 protein

A novel CRISPR-Cas enzyme was identified by the inventors through analysis of a metagenome of an uncultured sample, deduplication, and protein clustering analysis. Blast results show that the Cas protein has a low sequence identity with the Cas proteins previously reported. The Cas protein is designated as Cas-sf6728 in the present disclosure. An amino acid sequence of the Cas-sf6728 protein is shown in SEQ ID No. 1, and a nucleotide sequence encoding the Cas-sf6728 protein is shown in SEQ ID No. 2. A pairing region sequence in crRNA that targets tracrRNA is shown in SEQ ID No. 3, and a sequence of the tracrRNA is shown in SEQ ID No. 4.
Amino acid sequence of Cas-sf6728 (SEQ ID No. 1): 
MADESTKPKTHIRTLKLKLKVVGDDPKTSWKRLRQTSNDTWRAANWIAAGQFMN
DQLVRRLYARLKVDSKDPAAVQKVEDDFKAVFGTKRQATTERDIKQQFPDLPPCVTNTL
NQFVVASYTKEKPDMLAGNRSLRTYRKGLPIPTSRASVDFSAQDGRHIVSWKVQRGEQI
QFEVFYGRDKAGNERTIGLIIATERDYGAPSIQLNEKGLFLLLPVKEPAQDATLDPKRVVG
VDLGLAVPAYAAVSDGPARRPMGSAEDFLKTRLQMQSRRRRLQRSLVAVHGGNGRQRK
MKAMDRLSEKERHFARTYNHMISRRLVDFALQVGAGQINMELLEGFGRDEQQAFVLRN
WSFFELQQLIGEKAARVGIKVRHVDPYHTSQICSECGNWAQGQRDGRDFVCQNCGLKL
DADYNAAVNISRSTKYVQRKEQCRAYIERQTTTTESDDIEKSETDSAGPTAEG*.
Nucleotide sequence for Cas-sf6728 (SEQ ID No. 2): 
atggccgatgaaagcaccaaaccgaaaacccatattcgcaccctgaaactgaaactgaaggttgtgggtgatgatccgaaaaccag
ctggaaacgtctgcgtcagaccagcaatgatacctggcgtgcagcaaattggattgccgccggtcagtttatgaatgatcagctggtgcgtc
gtctgtatgcacgtctgaaagttgatagcaaagatccggccgccgtgcagaaagttgaagatgattttaaagccgttttcggtaccaaacgcc
aggcaaccaccgaacgtgatattaaacagcagtttccggatctgccgccgtgtgtgaccaataccctgaatcagtttgtggttgcaagttatac
caaagaaaaaccggatatgctggcaggcaatcgcagcctgcgtacctatcgcaaaggtctgccgattccgaccagccgcgccagtgtgg
attttagcgcacaggatggtcgtcatattgttagctggaaagttcagcgcggcgaacagattcagtttgaagttttttatggccgtgataaagca
 
ggcaatgaacgcaccattggtctgattattgcaaccgaacgtgactatggtgcacctagtattcagctgaatgaaaaaggcctgtttctgctgc
tgccggttaaagaaccggcccaggatgcaaccctggaccctaaacgcgtggttggtgttgatctgggtctggccgtgccggcatatgccgc
cgtgagcgatggcccggcccgtcgtcctatgggtagtgcagaagattttctgaaaacccgtctgcagatgcagagccgccgccgccgtct
gcagcgtagtctggttgccgtgcatggtggcaatggccgtcagcgtaaaatgaaagccatggatcgtctgagtgaaaaagaacgtcattttg
cccgcacctataatcatatgattagtcgccgcctggtggattttgccctgcaggttggtgccggtcagattaatatggaactgctggaaggctt
tggccgtgatgaacagcaggcctttgttctgcgcaattggagcttttttgaactgcagcagctgattggtgaaaaagcagcacgtgtgggtatt
aaagttcgtcatgtggacccttatcataccagccagatttgcagtgaatgtggcaattgggcacagggccagcgcgatggccgtgattttgtg
tgccagaattgcggtctgaaactggatgcagattataatgcagcagtgaatattagccgtagcaccaaatatgtgcagcgtaaagaacagtgt
cgtgcctatattgaacgccagaccaccaccaccgaaagcgatgatattgaaaaaagtgaaaccgatagcgcaggcccgaccgcagaagg
ctaa.
Pairing region sequence in crRNA that targets tracrRNA for Cas-sf6728 (SEQ ID No. 3):
   AACGAGCCCACGCGUGCCGGGAUGGGUCG.
Sequence of tracrRNA for Cas-sf6728 (SEQ ID No. 4):

UCAACCAAAUAUGUCCAACGAAAGGAGCAAUGCCGGGCCUACAUCGAGCGAC
AAACCACAACAACCGAAUCCGACGAUAUCGAGAAGUCGGAAACUGAUUCGGCUGG
GCCCACAGCCGAAGGGUGAGGGCUAAGCGCCACUCACCAACCCCGCAUGCGCAAU
AUAUUGCGACUUGGCUCGUCCCACAGCACAACAUGGCA.

### Example 2 PAM identification for the Cas-sf6728 protein

Construction of an expression plasmid for the Cas-sf6728 protein in Example 1: The nucleotide sequence for Cas-sf6728 was subjected to codon optimization for *Escherichia coli* (*E. coli*)*,* then synthesized, and ligated to an *E. coli* expression vector PeT28(a)+. In addition, a J23119 promoter was added to drive the transcription of tracrRNA and crRNA for the Cas protein to produce a vector PeT28(a)+-Cas-J23119-gRNA. A gRNA sequence is as follows: TCAACCAAATATGTCCAACGAAAGGAGCAATGCCGGGCCTACATCGAGCGACAAACC ACAACAACCGAATCCGACGATATCGAGAAGTCGGAAACTGATTCGGCTGGGCCCACA GCCGAAGGGTGAGGGCTAAGCGCCACTCACCAACCCCGCATGCGCAATATATTGCGA CTTGGCTCGTCCCACAGCACAACATGGCAGAAAAACGAGCCCACGCGTGCCGGGAT GGGTCGTCCCCTACGTGCTGCTGAAGTTGC, where an underlined sequence represents a target sequence.

Construction of a PAM library: The following sequence was synthesized: CGTGTTTCGTAAAGTCTGGAAACGCGGAAGCCCCCAGCGCTTCAGCGTTCNNNNNNT CCCCTACGTGCTGCTGAAGTTGCCCGCAA, where N represents a random deoxynucleotide and an underlined sequence represents a target sequence. The sequence was subjected to a fill-in reaction with a Klenow enzyme, and then ligated to a pacyc184 vector. A ligation product was transformed into *E. coli.* Then, plasmid extraction was conducted to produce the PAM library.

PAM library depletion assay: The expression vector PeT28(a)+-Cas-J23119-gRNA and the PAM library plasmid were co-transformed into competent BL21 (DE3) cells. Transformed cells were inoculated on an LB plate including kanamycin and chloromycin, and cultured overnight at 37°C. Resulting cells were collected, inoculated, and adjusted to a concentration corresponding to OD600 of 0.6 to 0.8. Isopropyl-β-D-thiogalactoside (IPTG) was added at 0.2 mM, and induction was conducted for 4 h at 37°C. Plasmid extraction was conducted with a FastPure EndoFree Plasmid Maxi Kit (vazyme) to produce a depleted PAM library. Primers: PAM-F: GGTCTTCGGTTTCCGTGTT; and PAM-R: TGGCGTTGACTCTCAGTCAT. With 30 ng/µL of a plasmid (PAM library) as a template, polymerase chain reaction (PCR) amplification was conducted to produce a sample of a control group. With 30 ng/µL of a plasmid (depleted PAM library) as a template, PCR amplification was conducted to produce a sample of an experimental group. The sample of the control group and the sample of the experimental group were subjected to next-generation sequencing for data analysis.

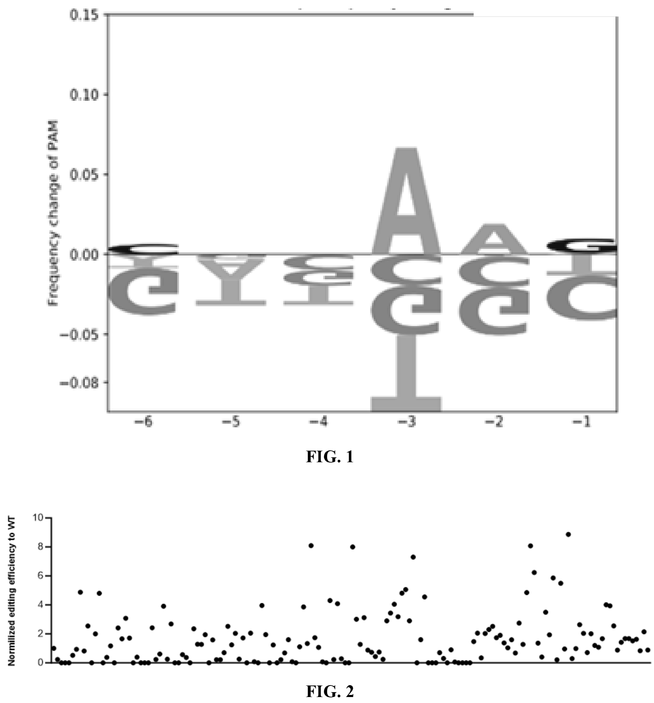

The PAM preference of the Cas-sf6728 protein was obtained, as shown in FIG. 1. The PAM preference of the Cas-sf6728 protein is 5'-AAN-3', where N represents A/C/G/T.

### Example 3 Editing efficiency of the Cas-sf6728 protein in animal cells

A gene-editing activity of the Cas-sf6728 protein was validated in the animal cells. A vector pcDNA3.3 was modified to carry an enhanced cyan fluorescent protein (ECFP) gene. A fusion protein SV40 NLS-Cas-NLS was inserted through an enzyme cleavage site BsmB1, and a U6 promoter and a gRNA sequence were inserted through an enzyme cleavage site Mfe1. A gRNA sequence is as follows: TCAACCAAATATGTCCAACGAAAGGAGCAATGCCGGGCCTACATCGAGCGACAAACC ACAACAACCGAATCCGACGATATCGAGAAGTCGGAAACTGATTCGGCTGGGCCCACA GCCGAAGGGTGAGGGCTAAGCGCCACTCACCAACCCCGCATGCGCAATATATTGCGA CTTGGCTCGTCCCACAGCACAACATGGCAGAAAAACGAGCCCACGCGTGCCGGGAT GGGTCGTCCCCTACGTGCTGCTGAAGTTGC, where an underlined sequence represents a target sequence. A CMV promoter was used to initiate the expression of a fusion protein SV40 NLS-Cas-NLS-ECFP. A protein Cas-NLS was linked to ECFP through a linker peptide T2A. After a pUC19 vector was modified, the expression of a tdTomato-T2A-GF(5 spacer1 24bp)FP gene was initiated by a promoter EF-1α. After a target site 5 spacer1 24bp was recognized and edited by the Cas protein, an editing efficiency of the Cas protein was analyzed by determining a proportion of green fluorescent protein (GFP)-positive cells among cyan fluorescent protein (CFP)/tdTomato-double-positive cells.

Plating: 293T cells growing to a confluency of 70% to 80% were collected and inoculated into a 12-well plate at 8 * 10^4 cells/well.

Transfection: 12 h to 24 h after the plating, transfection was conducted. 2 µg of a plasmid was thoroughly mixed with 100 µL of opti-MEM, then 4 µL of TransIntro^{®} EL Transfection Reagent (TRAN) was added, and incubation was conducted at room temperature for 15 min to 20 min. A mixed solution produced after the incubation was added to a medium with cells growing for transfection. 24 h after the transfection, the original medium was replaced with a normal medium. 48 h after the transfection, flow cytometry analysis was performed. According to analysis results, the editing efficiency of the Cas-sf6728 protein is 5.89%.

### Example 4 Acquisition of mutant Cas-sf6728 proteins

On the basis of Cas-sf6728 (with an amino acid sequence shown in SEQ ID No. 1), potential amino acids of the Cas enzyme to bind to a target sequence were subjected to site-directed mutagenesis through bioinformatics methods. A variant of the Cas protein was produced through PCR-based site-directed mutagenesis, which could be conducted by a common site-directed mutagenesis method in the art. A specific method was as follows: The DNA sequence for Cas-sf6728 was designed into two parts with a mutation site as a center. Two pairs of primers were designed to amplify the two parts of the DNA sequence, respectively. A sequence to be mutated was introduced into the primers. A combination of mutants was constructed by splitting DNA into a plurality of segments and then conducting PCR and Gibson clone. TransStart FastPfu DNA Polymerase (including 2.5 mM dNTPs) was adopted as a fragment amplification kit. A specific experimental procedure was detailed in the manual. FastPure^{®} Gel DNA Extraction Mini Kit was adopted as a gel recovery kit. A specific experimental procedure was detailed in the manual. pEASY-Basic Seamless Cloning and Assembly Kit (CU201-03) was adopted as a kit for vector construction. A specific experimental procedure was detailed in the manual.

In this example, based on SEQ ID No. 1, a mutation was introduced at the following sites: T36W, D39L, L65R, S69R, A73R, V75R, A119R, T122R, N132R, S154R, A155R, Q156R, D157R, Q171R, E186R, L191R, T195R, E208R, T264R, S278R, A281R, D296R, H304R, Q342R, A344R, and other amino acid sites. Based on the aforementioned amino acid mutation sites, single-mutant Cas-sf6728 proteins T36W, D39L, L65R, S69R, A73R, V75R, A119R, T122R, N132R, S154R, A155R, Q156R, D157R, Q171R, E186R, L191R, T195R, E208R, T264R, S278R, A281R, D296R, H304R, Q342R, and A344R and a multi-mutant Cas-sf6728 protein L65R-V75R were produced. The single-mutant Cas-sf6728 proteins were produced based on SEQ ID No. 1 through the following mutations, respectively: counting from an N terminus, a mutation of a 36th amino acid to W, a mutation of a 39th amino acid to L, a mutation of a 65th amino acid to R, a mutation of a 69th amino acid to R, a mutation of a 73rd amino acid to R, a mutation of a 75th amino acid to R, a mutation of a 119th amino acid to R, a mutation of a 122nd amino acid to R, a mutation of a 132nd amino acid to R, a mutation of a 154th amino acid to R, a mutation of a 155th amino acid to R, a mutation of a 156th amino acid to R, a mutation of a 157th amino acid to R, a mutation of a 171st amino acid to R, a mutation of a 186th amino acid to R, a mutation of a 191st amino acid to R, a mutation of a 195th amino acid to R, a mutation of a 208th amino acid to R, a mutation of a 264th amino acid to R, a mutation of a 278th amino acid to R, a mutation of a 281st amino acid to R, a mutation of a 296th amino acid to R, a mutation of a 304th amino acid to R, a mutation of a 342nd amino acid to R, and a mutation of a 344th amino acid to R. The multi-mutant Cas-sf6728 protein L65R-V75R was produced based on SEQ ID No. 1 through the following mutations: counting from the N terminus, a mutation of a 65th amino acid to R and a mutation of a 75th amino acid to R.

### Example 5 Verification of editing activities of mutant Cas-sf6728 proteins

Gene-editing activities of the single-mutant Cas-sf6728 proteins obtained in Example 4 were verified in cells, with a WT Cas-sf6728 protein (SEQ ID No. 1) as a control. A target site cagcctagctcaggagaagt on a transthyretin (TTR) gene was selected and transformed into cells, and then an editing efficiency was tested.

A vector pcDNA3.3 was modified to carry an enhanced green fluorescent protein (EGFP). A fusion protein SV40 NLS-Cas was inserted through enzyme cleavage sites XbaI and PstI, and a U6 promoter and a gRNA sequence were inserted through an enzyme cleavage site Mfe1. A CMV promoter was used to initiate the expression of a fusion protein SV40 NLS-Cas-XX-NLS-GFP. A protein Cas-XX-NLS was linked to GFP through a linker peptide T2A. Plating: Cells growing to a confluency of 70% to 80% were collected and inoculated into a 12-well plate at 8 * 10^4 cells/well. Transfection: 24 h after the plating, transfection was conducted. 6.25 µL of a Hieff Trans^{™} lipofection nucleic acid transfection reagent and 100 µL of opti-MEM were thoroughly mixed to produce a diluted Hieff Trans^{™} lipofection nucleic acid transfection reagent. 2.5 µg of a plasmid and 100 µL of opti-MEM were thoroughly mixed to produce a diluted plasmid. The diluted Hieff Trans^{™} lipofection nucleic acid transfection reagent was thoroughly mixed with the diluted plasmid, and incubation was conducted at room temperature for 20 min. A mixed solution produced after the incubation was added to a medium with cells growing for transfection. 48 h after the transfection, digestion was conducted with trypsin-ethylenediaminetetraacetic acid (EDTA) (0.05%), and cells with a GFP signal were sorted by fluorescence-activated cell sorting (FACS).

DNA was extracted, and a region near an editing region was amplified by PCR and sent for hiTOM sequencing: Cells were digested with trypsin, then collected, and subjected to genomic DNA extraction by a cell/tissue genomic DNA extraction kit (Bioteke). A region near a target site on the extracted genomic DNA was amplified. A target sequence was amplified using sequencing primers: TTR-NGS-F1: CTATTCGcaggtttgcagtcagattgg, and TTR-NGS-R1: ATCACGgtaggaatgggatgtcacag. A PCR product was subjected to hiTOM sequencing. Sequencing data was analyzed. With an editing efficiency of the WT Cas-sf6728 protein (SEQ ID No. 1) as a baseline (100%), editing efficiencies of different single-mutant Cas-sf6728 proteins for the target site were determined, as shown in FIG. 2. Single-mutant Cas-sf6728 proteins with significantly-improved editing efficiencies were selected, and editing efficiencies of these single-mutant Cas-sf6728 proteins were summarized in the following table.

| Mutant protein name | Editing efficiency relative to the WT Cas-sf6728 protein (%) |
|---|---|
| T36W | 260 |
| D39L | 330 |
| L65R | 425 |
| S69R | 255 |
| A73R | 200 |
| V75R | 280 |
| A119R | 485 |
| T122R | 808 |
| N132R | 624 |
| S154R | 241 |
| A155R | 166 |
| Q156R | 308 |
| D157R | 172 |
| Q171R | 242 |
| E186R | 349 |
| L191R | 586 |
| T195R | 549 |
| E208R | 887 |
| T264R | 731 |
| S278R | 392 |
| A281R | 268 |
| D296R | 236 |
| H304R | 194 |
| Q342R | 401 |
| A344R | 394 |

According to the above results, a 36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, or 344th amino acid from an N terminus of SEQ ID No. 1 is a key site for the activity of the Cas-sf6728 protein. Mutations at these amino acid sites can significantly enhance the editing efficiency of the Cas-sf6728 protein.

Further, an editing efficiency of the multi-mutant Cas-sf6728 protein L65R-V75R was validated using the method described above. According to verification results, an editing efficiency of the multi-mutant Cas-sf6728 protein L65R-V75R is approximately 7-fold higher than an editing efficiency of the WT Cas-sf6728 protein.

### Example 6 Modification of gRNA for the Cas-sf6728 protein

The gRNA for the Cas-sf6728 protein includes tracrRNA and crRNA. The tracrRNA can pair with a pairing region of the crRNA to produce a duplex. The crRNA further includes a region that hybridizes with a target sequence (namely, a targeting sequence for a target nucleic acid). In this example, a non-targeting sequence portion of the gRNA was modified. A non-targeting sequence of WT gRNA for the Cas-sf6728 protein is shown in SEQ ID No. 5.

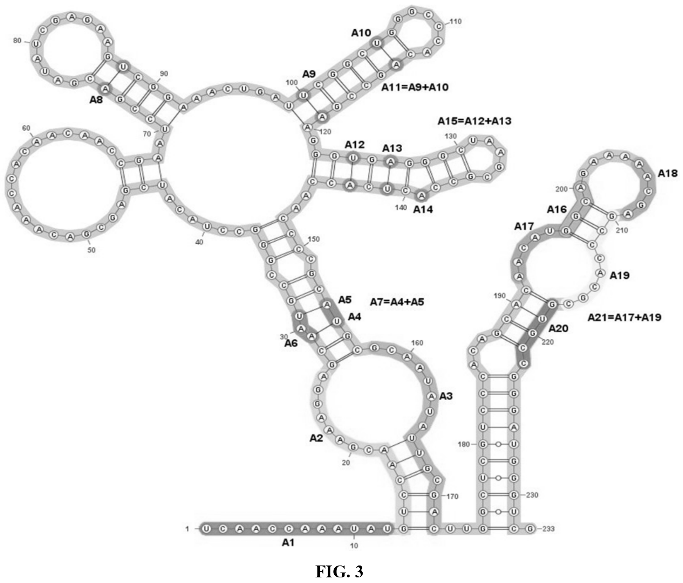

A secondary structure of the non-targeting sequence portion of the WT gRNA for the Cas-sf6728 protein was predicted using RNAfold2, as shown in FIG. 3. Based on the analysis of characteristics of the secondary structure, nucleotides at different positions were selected for truncation or mutation. Modified non-targeting sequence portions of gRNA are shown in the following table.

Editing efficiencies of editing systems produced from the WT Cas-sf6728 protein and the modified gRNAs were assessed using a fluorescence reporter system. The fluorescent reporter system for validating an editing efficiency of a Cas-sf6728 editing system was constructed with reference to the literature (Yang, Yi, et al. "Highly efficient and rapid detection of the cleavage activity of Cas9/gRNA via a fluorescent reporter." Applied biochemistry and biotechnology 180.4 (2016): 655-667.). In this fluorescent reporter system, a fluorescent reporter vector carried a red fluorescent protein (RFP) and non-luminous GFFP, and a Cas vector carried CFP. A target site AAGTTTAACAGTGGCCTTATTAA on a GFFP sequence was selected for testing, where an underlined part was a PAM sequence. 48 h after 293T cells were transfected, a proportion of GFP-positive cells in a CFP- and RFP-positive cell population was determined by FACS to determine an editing efficiency. The editing efficiencies of the editing systems produced from the WT Cas-sf6728 protein and the modified gRNAs are shown in the following table.

| Non-targeting sequence of gRNA | Sequence information | Mutation type relative to WT | SEQ ID No. | Editing efficiency/ % |
|---|---|---|---|---|
| WT | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatggcagaaaaacgagcccacgcgtgccggg atgggtcg | None | 5 | 5.99 |
| A1 | gtccaacgaaaggagcaatgccgggcctacatcgagc gacaaaccacaacaaccgaatccgacgatatcgagaa gtcggaaactgattcggctgggcccacagccgaaggg tgagggctaagcgccactcaccaaccccgcatgcgca atatattgcgacttggctcgtcccacagcacaacatggc agaaaaacgagcccacgcgtgccgggatgggtcg | Deletion of 1st to 12th nucleotides | 6 | 25.15 |
| A2 | gcaatgccgggcctacatcgagcgacaaaccacaaca accgaatccgacgatatcgagaagtcggaaactgattc ggctgggcccacagccgaagggtgagggctaagcgc cactcaccaaccccgcatgcgcaatatattgcgacttgg ctcgtcccacagcacaacatggcagaaaaacgagccc acgcgtgccgggatgggtcg | Deletion of 1st to 26th nucleotides | 7 | 21.33 |
| A3 | tcaaccaaatatgcaatgccgggcctacatcgagcgac aaaccacaacaaccgaatccgacgatatcgagaagtcg gaaactgattcggctgggcccacagccgaagggtgag ggctaagcgccactcaccaaccccgcatgcttggctcg tcccacagcacaacatggcagaaaaacgagcccacgc gtgccgggatgggtcg | Deletion of 13th to 26th and 158th to 172nd nucleotides | 8 | 22.17 |
| A4 | tcaaccaaatatgtccaacgaaaggagccatgccgggc ctacatcgagcgacaaaccacaacaaccgaatccgac gatatcgagaagtcggaaactgattcggctgggcccac agccgaagggtgagggctaagcgccactcaccaaccc cgcaggcgcaatatattgcgacttggctcgtcccacagc acaacatggcagaaaaacgagcccacgcgtgccggg atgggtcg | Substitution of A with C at position 29 and substitution of U with G at position 155 | 9 | 14.69 |
| A5 | tcaaccaaatatgtccaacgaaaggagcaacgccggg cctacatcgagcgacaaaccacaacaaccgaatccga cgatatcgagaagtcggaaactgattcggctgggccca cagccgaagggtgagggctaagcgccactcaccaacc ccgcgtgcgcaatatattgcgacttggctcgtcccacag cacaacatggcagaaaaacgagcccacgcgtgccgg gatgggtcg | Substitution of U with C at position 31 and substitution of A with G at position 154 | 10 | 23.13 |
| A6 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcattgcgcaatatattgcgacttggctcgtcccacagc acaacatggcagaaaaacgagcccacgcgtgccggg atgggtcg | Addition of U between U at position 155 and G at position 156 | 11 | 24.24 |
| A7 | tcaaccaaatatgtccaacgaaaggagccacgccggg cctacatcgagcgacaaaccacaacaaccgaatccga cgatatcgagaagtcggaaactgattcggctgggccca cagccgaagggtgagggctaagcgccactcaccaacc ccgcgggcgcaatatattgcgacttggctcgtcccacag cacaacatggcagaaaaacgagcccacgcgtgccgg gatgggtcg | A4 + A5 (substitution of A with C at position 29, substitution of U with C at position 31, substitution of A with G at position 154, and substitution of U with G at position 155) | 12 | 10.72 |
| A8 | tcaaccaaatatgtccaacgaaaggagcaatgccgggc ctacatcgagcgacaaaccacaacaaccgaatccgcc gatatcgagaaggcggaaactgattcggctgggcccac agccgaagggtgagggctaagcgccactcaccaaccc cgcatgcgcaatatattgcgacttggctcgtcccacagc acaacatggcagaaaaacgagcccacgcgtgccggg atgggtcg | Substitution of A with C at position 74 and substitution of U with G at position 88 | 13 | 16.22 |
| A9 | tcaaccaaatatgtccaacgaaaggagcaatgccgggc ctacatcgagcgacaaaccacaacaaccgaatccgac gatatcgagaagtcggaaactgatccggctgggcccac agccggagggtgagggctaagcgccactcaccaaccc cgcatgcgcaatatattgcgacttggctcgtcccacagc acaacatggcagaaaaacgagcccacgcgtgccggg atgggtcg | Substitution of U with C at position 100 and substitution of A with G at position 119 | 14 | 19.92 |
| A10 | tcaaccaaatatgtccaacgaaaggagcaatgccgggc ctacatcgagcgacaaaccacaacaaccgaatccgac gatatcgagaagtcggaaactgattcggccgggcccac ggccgaagggtgagggctaagcgccactcaccaaccc cgcatgcgcaatatattgcgacttggctcgtcccacagc acaacatggcagaaaaacgagcccacgcgtgccggg atgggtcg | Substitution of U with C at position 105 and substitution of A with G at position 114 | 15 | 21.12 |
| A11 | tcaaccaaatatgtccaacgaaaggagcaatgccgggc ctacatcgagcgacaaaccacaacaaccgaatccgac gatatcgagaagtcggaaactgatccggccgggccca cggccggagggtgagggctaagcgccactcaccaac cccgcatgcgcaatatattgcgacttggctcgtcccaca gcacaacatggcagaaaaacgagcccacgcgtgccg ggatgggtcg | A9 + A10 (substitution of U with C at position 100, substitution of U with C at position 105, substitution of A with G at position 114, and substitution of A with G at position 119) | 16 | 18.39 |
| A12 | tcaaccaaatatgtccaacgaaaggagcaatgccgggc ctacatcgagcgacaaaccacaacaaccgaatccgac gatatcgagaagtcggaaactgattcggctgggcccac agccgaagggggagggctaagcgccactccccaacc ccgcatgcgcaatatattgcgacttggctcgtcccacag cacaacatggcagaaaaacgagcccacgcgtgccgg gatgggtcg | Substitution of U with G at position 124 and substitution of A with C at position 143 | 17 | 22.57 |
| A13 | tcaaccaaatatgtccaacgaaaggagcaatgccgggc ctacatcgagcgacaaaccacaacaaccgaatccgac gatatcgagaagtcggaaactgattcggctgggcccac agccgaagggtgggggctaagcgccacccaccaacc ccgcatgcgcaatatattgcgacttggctcgtcccacag cacaacatggcagaaaaacgagcccacgcgtgccgg gatgggtcg | Substitution of A with G at position 126 and substitution of U with C at position 141 | 18 | 31.7 |
| A14 | tcaaccaaatatgtccaacgaaaggagcaatgccgggc ctacatcgagcgacaaaccacaacaaccgaatccgac gatatcgagaagtcggaaactgattcggctgggcccac agccgaagggtgagtggctaagcgccactcaccaacc ccgcatgcgcaatatattgcgacttggctcgtcccacag cacaacatggcagaaaaacgagcccacgcgtgccgg gatgggtcg | Addition of U between G at position 127 and G at position 128 | 19 | 19.32 |
| A15 | tcaaccaaatatgtccaacgaaaggagcaatgccgggc ctacatcgagcgacaaaccacaacaaccgaatccgac gatatcgagaagtcggaaactgattcggctgggcccac agccgaagggggggggctaagcgccaccccccaacc ccgcatgcgcaatatattgcgacttggctcgtcccacag cacaacatggcagaaaaacgagcccacgcgtgccgg gatgggtcg | A12 + A13 (substitutio n of U with G at position 124, substitution of A with G at position 126, substitution of U with C at position 141, and substitution of A with C at position 143) | 20 | 20.79 |
| A16 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatgaaaaacgagcccacgcgtgccgggatggg tcg | Deletion of 198th to 201st nucleotides | 21 | 12.92 |
| A17 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acgaaaaacgagcccacgcgtgccgggatgggtcg | Deletion of 192nd to 200th nucleotides | 22 | 21.13 |
| A18 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatggcagaaagcccacgcgtgccgggatgggt cg | Deletion of 205th to 209th nucleotides | 23 | 17.33 |
| A19 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatggcagaaagtgccgggatgggtcg | Deletion of 205th to 217th nucleotides | 24 | 21.44 |
| A20 | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatggcagaaagggatgggtcg | Deletion of 205th to 222nd nucleotides | 25 | 19.47 |

According to the experimental results, the modified gRNAs A1 to A20 all can significantly enhance the editing efficiency, and particularly, A13 (substitution of A with G at position 126 and substitution of U with C at position 141) demonstrates the optimal effect.

Subsequently, the modified gRNA sequences A1 to A20 were combined to produce new gRNA sequences. With the method described in Example 5, editing activities of editing systems produced from the combined gRNA sequences and the WT Cas-sf6728 protein were tested *in vivo.* Results are shown in the following table. According to these results, the combined gRNA sequence of A3 + A13 leads to the optimal effect, and enables an editing activity 5-fold or more higher than an editing activity of the WT gRNA.

| gRNA combination | Sequence information | Mutation type relative to WT | SEQ ID No. | Editing efficiency/ % |
|---|---|---|---|---|
| WT | tcaaccaaatatgtccaacgaaaggagcaatgccgggc ctacatcgagcgacaaaccacaacaaccgaatccgac gatatcgagaagtcggaaactgattcggctgggcccac agccgaagggtgagggctaagcgccactcaccaaccc cgcatgcgcaatatattgcgacttggctcgtcccacagc acaacatggcagaaaaacgagcccacgcgtgccggg atgggtcg | None | 5 | 0.86 |
| A1+A3 | gcaatgccgggcctacatcgagcgacaaaccacaaca accgaatccgacgatatcgagaagtcggaaactgattc ggctgggcccacagccgaagggtgagggctaagcgc cactcaccaaccccgcatgcttggctcgtcccacagca caacatggcagaaaaacgagcccacgcgtgccgggat gggtcg | Deletion of 1st to 26th and 158th to 172nd nucleotides | 26 | 2.71 |
| A1+A13 | gtccaacgaaaggagcaatgccgggcctacatcgagc gacaaaccacaacaaccgaatccgacgatatcgagaa gtcggaaactgattcggctgggcccacagccgaaggg tgggggctaagcgccacccaccaaccccgcatgcgca atatattgcgacttggctcgtcccacagcacaacatggc agaaaaacgagcccacgcgtgccgggatgggtcg | Deletion of 1st to 12th nucleotides , substitution of A with G at position 126, and substitution of U with C at position 141 | 27 | 4.22 |
| A3+A13 | tcaaccaaatatgcaatgccgggcctacatcgagcgac aaaccacaacaaccgaatccgacgatatcgagaagtcg gaaactgattcggctgggcccacagccgaagggtggg ggctaagcgccacccaccaaccccgcatgcttggctcg tcccacagcacaacatggcagaaaaacgagcccacgc gtgccgggatgggtcg | Deletion of 13th to 26th and 158th to 172nd nucleotides , substitution of A with G at position 126, and substitution of U with C at position 141 | 28 | 5.83 |
| A5+A6 | tcaaccaaatatgtccaacgaaaggagcaacgccggg cctacatcgagcgacaaaccacaacaaccgaatccga cgatatcgagaagtcggaaactgattcggctgggccca cagccgaagggtgagggctaagcgccactcaccaacc ccgcgttgcgcaatatattgcgacttggctcgtcccaca gcacaacatggcagaaaaacgagcccacgcgtgccg ggatgggtcg | Substitution of U with C at position 31, substitution of A with G at position 154, and addition of U between U at position 155 and G at position 156 | 29 | 2.63 |
| A5+A13 | tcaaccaaatatgtccaacgaaaggagcaacgccggg | Substitutio | 30 | 1.43 |
| | cctacatcgagcgacaaaccacaacaaccgaatccga cgatatcgagaagtcggaaactgattcggctgggccca cagccgaagggtgggggctaagcgccacccaccaac cccgcgtgcgcaatatattgcgacttggctcgtcccaca gcacaacatggcagaaaaacgagcccacgcgtgccg ggatgggtcg | n of U with C at position 31, substitution of A with G at position 154, substitution of A with G at position 126, and substitution of U with C at position 141. | | |
| A13+A17 | tcaaccaaatatgtccaacgaaaggagcaatgccgggc ctacatcgagcgacaaaccacaacaaccgaatccgac gatatcgagaagtcggaaactgattcggctgggcccac agccgaagggtgggggctaagcgccacccaccaacc ccgcatgcgcaatatattgcgacttggctcgtcccacag cacgaaaaacgagcccacgcgtgccgggatgggtcg | Substitution of A with G at position 126, substitution of U with C at position 141, and deletion of 192nd to 200th nucleotides | 31 | 1.89 |

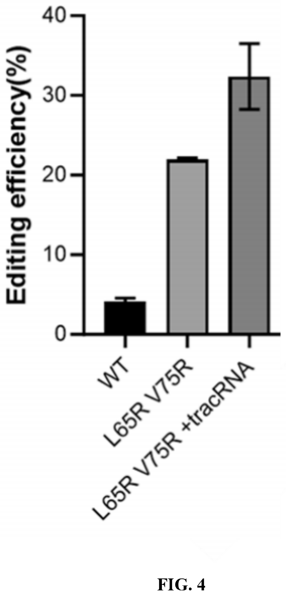

Further, editing efficiencies of an editing system of WT Cas-sf6728 protein + WT gRNA sequence (WT in FIG. 4), an editing system of multi-mutant Cas-sf6728 protein L65R-V75R + WT gRNA sequence (L65R V75R in FIG. 4), and an editing system of multi-mutant Cas-sf6728 protein L65R-V75R + modified gRNA sequence (A3 + A13) (L65R V75R + tracrRNA in FIG. 4) were detected by the method in Example 5. Results are shown in FIG. 4. Compared to the editing system of WT Cas-sf6728 protein + WT gRNA sequence, the editing system of multi-mutant Cas-sf6728 protein L65R-V75R + WT gRNA sequence demonstrates an *in vivo* editing activity increasing approximately 7-fold, and the editing system of multi-mutant Cas-sf6728 protein L65R-V75R + modified gRNA sequence (A3 + A13) demonstrates an *in vivo* editing activity increasing approximately 8-fold to 33%. It can be seen that both a mutation for the Cas-sf6728 protein and a modification for gRNA can significantly enhance the editing activity of a Cas-sf6728 editing system.

Although the specific implementations of the present disclosure have been described in detail, those skilled in the art will understand that various modifications and changes can be made to the details according to all teachings published, and such modifications and changes are all within the protection scope of the present disclosure. The full content of the present disclosure is defined by the appended claims and any equivalents thereof.

## Claims

1. A clustered regularly interspaced short palindromic repeat (CRISPR)-associated (Cas) protein, wherein the Cas protein is any one of the following I to IV:
I, a Cas protein that has an amino acid sequence possessing a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with SEQ ID No. 1 and basically retains a biological function of SEQ ID No. 1;
II, a Cas protein that has an amino acid sequence comprising substitution, deletion, or addition of one or more amino acids (comprising 1, 2, 3, 4, 5, 6, 7, 8, 9, or 10 amino acids) relative to SEQ ID No. 1 and basically retains the biological function of SEQ ID No. 1;
III, a Cas protein comprising the amino acid sequence shown in SEQ ID No. 1; and
IV, a Cas protein that has an amino acid sequence comprising a mutation of any one or more of36th, 39th, 65th, 69th, 73rd, 75th, 119th, 122nd, 132nd, 154th, 155th, 156th, 157th, 171st, 186th, 191st, 195th, 208th, 264th, 278th, 281st, 296th, 304th, 342nd, and 344th amino acids relative to the amino acid sequence shown in SEQ ID No. 1.

2. A fusion protein, comprising the Cas protein according to claim 1 and other modification moiety.

3. An isolated polynucleotide, wherein the polynucleotide is a polynucleotide sequence encoding the Cas protein according to claim 1 or a polynucleotide sequence encoding a fusion protein according to claim 2.

4. A vector, comprising the polynucleotide according to claim 3 and a regulatory element operably linked thereto.

5. A guide RNA (gRNA), comprising a targeting sequence and a non-targeting sequence, wherein the non-targeting sequence comprises a trans-activating CRISPR RNA (tracrRNA) and a pairing region sequence in a CRISPR RNA (crRNA) that targets the tracrRNA; and
preferably, the non-targeting sequence of the gRNA is shown in SEQ ID No. 5; or the non-targeting sequence of the gRNA comprises a base mutation relative to SEQ ID No. 5, wherein the base mutation is selected from any one or more (comprising any 1, 2, 3, 4, or 5) of the following (1) to (20):
(1) deletion of 1st to 12th bases in SEQ ID No. 5;
(2) deletion of 1st to 26th bases in SEQ ID No. 5;
(3) deletion of 13th to 26th and 158th to 172nd bases in SEQ ID No. 5;
(4) substitution of A with C at position 29 and substitution of U with G at position 155 in SEQ ID No. 5;
(5) substitution of U with C at position 31 and substitution of A with G at position 154 in SEQ ID No. 5;
(6) addition of U between U at position 155 and G at position 156 in SEQ ID No. 5;
(7) substitution of A with C at position 29, substitution of U with C at position 31, substitution of A with G at position 154, and substitution of U with G at position 155 in SEQ ID No. 5;
(8) substitution of A with C at position 74 and substitution of U with G at position 88 in SEQ ID No. 5;
(9) substitution of U with C at position 100 and substitution of A with G at position 119 in SEQ ID No. 5;
(10) substitution of U with C at position 105 and substitution of A with G at position 114 in SEQ ID No. 5;
(11) substitution of U with C at position 100, substitution of U with C at position 105, substitution of A with G at position 114, and substitution of A with G at position 119 in SEQ ID No. 5;
(12) substitution of U with G at position 124 and substitution of A with C at position 143 in SEQ ID No. 5;
(13) substitution of A with G at position 126 and substitution of U with C at position 141 in SEQ ID No. 5;
(14) addition of U between G at position 127 and G at position 128 in SEQ ID No. 5;
(15) substitution of U with G at position 124, substitution of A with G at position 126, substitution of U with C at position 141, and substitution of A with C at position 143 in SEQ ID No. 5;
(16) deletion of 198th to 201st bases in SEQ ID No. 5;
(17) deletion of 192nd to 200th bases in SEQ ID No. 5;
(18) deletion of 205th to 209th bases in SEQ ID No. 5;
(19) deletion of 205th to 217th bases in SEQ ID No. 5; and
(20) deletion of 205th to 222nd bases in SEQ ID No. 5.

6. A CRISPR-Cas system, comprising the Cas protein according to claim 1 and at least one gRNA capable of binding to the Cas protein, wherein the gRNA comprises a region that binds to the Cas protein and a targeting sequence that targets a nucleic acid, or the gRNA is the gRNA according to claim 5.

7. A composition, comprising:
(i) a protein component selected from the Cas protein according to claim 1 or a fusion protein according to claim 2; and
(ii) a nucleic acid component selected from a gRNA or a nucleic acid encoding the gRNA or a precursor RNA of the gRNA or a nucleic acid encoding the precursor RNA of the gRNA, wherein the gRNA comprises a region that binds to the Cas protein according to claim 1 and a targeting sequence that targets a nucleic acid, or the gRNA is a gRNA according to claim 5,
wherein the protein component is capable of binding to the nucleic acid component to produce a complex.

8. An engineered host cell, comprising the Cas protein according to claim 1 or a fusion protein according to claim 2 or a polynucleotide according to claim 3 or a vector according to claim 4 or a gRNA according to claim 5 or a CRISPR-Cas system according to claim 6 or a composition according to claim 7.

9. A use of the Cas protein according to claim 1 or a fusion protein according to claim 2 or a polynucleotide according to claim 3 or a vector according to claim 4 or a gRNA according to claim 5 or a CRISPR-Cas system according to claim 6 or a composition according to claim 7 or a host cell according to claim 8 in gene editing, gene targeting, gene cleavage, cleavage of a double-stranded DNA, a single-stranded DNA, or a single-stranded RNA, non-specific cleavage and/or degradation of a branched nucleic acid, non-specific cleavage of a single-stranded nucleic acid, nucleic acid detection, specific editing of a double-stranded nucleic acid, base editing of the double-stranded nucleic acid, or base editing of the single-stranded nucleic acid; or
in preparation of a formulation or kit, wherein the formulation or kit is provided for the gene editing, the gene targeting, the gene cleavage, the cleavage of the double-stranded DNA, the single-stranded DNA, or the single-stranded RNA, the non-specific cleavage and/or degradation of the branched nucleic acid, the non-specific cleavage of the single-stranded nucleic acid, the nucleic acid detection, the specific editing of the double-stranded nucleic acid, the base editing of the double-stranded nucleic acid, or the base editing of the single-stranded nucleic acid.

10. A method for editing a target nucleic acid, targeting the target nucleic acid, or cleaving the target nucleic acid, comprising: allowing the target nucleic acid to be in contact with the Cas protein according to claim 1 or a fusion protein according to claim 2 or a polynucleotide according to claim 3 or a vector according to claim 4 or a gRNA according to claim 5 or a CRISPR-Cas system according to claim 6 or a composition according to claim 7 or a host cell according to claim 8.
